# EUROPEAN PATENT SPECIFICATION

(11) **EP 4 384 233 B1**
(45) Date of publication and mention of the grant of the patent: **15.07.2026**
(21) Application number: 22768474.3
(22) Date of filing: 23.08.2022
(51) Int. Cl.: A61M 1/00

(54) **FLUID STORAGE CANISTER**
KANISTER ZUR FLÜSSIGKEITSSPEICHERUNG
CARTOUCHE DE STOCKAGE DE FLUIDE

(30) Priority: 15.09.2021 US 202163244457 P
(43) Date of publication of application: 19.06.2024
(73) Proprietor: KCI Manufacturing Unlimited Company, Athlone, Co. Westmeath, N37XF22 (IE)
(72) Inventor: PRATT, Benjamin A., Bracknell Berkshire RG12 8HT (GB); EDWARDS, Thomas A., Bracknell Berkshire RG12 8HT (GB)
(74) Representative: Simmons & Simmons
(86) International application number: PCT/IB2022/057872
(87) International publication number: WO 2023/042013

(56) References cited:
- WO-A1-2022/195377
- US-A1- 2011 288 510
- US-A1- 2013 053 798
- US-A1- 2019 175 797

## Description

### CROSS-REFERENCE TO RELATED APPLICATIONS

This application claims the benefit of priority to U.S. Provisional Application No. 63/244,457, filed on September 15, 2021.

### TECHNICAL FIELD

The invention set forth in the appended claims relates generally to tissue treatment systems and more particularly, but without limitation, to an improved fluid canister structure.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.
US2013/0053798 discloses an inline storage-and-liquid processing pouch for use with body fluids form patient.
US2019/0175797 discloses a container including a containing housing formed at least in part by a liquid-impermeable, vapor-permeable material.
US2011/0288510 discloses a reduced-pressure treatment pump including a pump control unit fluidly separate from a collection unit.
WO2022/195377 discloses a canister having first and second compartments and an airflow pathway with a planar region and an inlet at an obtuse angle.

### BRIEF SUMMARY

The invention is defined by the independent claim. A selection of optional features of the invention is set out in the dependent claims.

### BRIEF DESCRIPTION OF THE DRAWINGS

Figure 1 is a functional block diagram of an example embodiment of a therapy system that can provide negative-pressure treatment in accordance with this specification;
Figure 2 is an assembled view of an example embodiment of a canister of the therapy system of Figure 1;
Figure 3 is an exploded view of the canister of Figure 2, illustrating additional details that may be associated with some embodiments;
Figure 4 is an exploded view of a portion of the canister of Figures 3, illustrating additional detail that may be associated with some embodiments;
Figure 5 is an assembled cross-sectional view of the portion of the canister of Figure 4;
Figure 6 is an exploded view of another example of the portion of the canister of Figure 3 having a support layer perforated with hexagonal holes that may be used with some embodiments;
Figure 7 is an exploded view illustrating a portion of an operation to assemble the portion of the canister of Figure 6;
Figure 8 is an exploded view illustrating a portion of another operation to assemble the portion of the canister of Figure 6;
Figure 9 is an exploded view of another example of the portion of the canister of Figure 3 having a nonwoven layer disposed between a support layer and an evaporative layer that may be associated with some embodiments;
Figure 10 is an assembled cross-sectional view of the portion of the canister of Figure 9;
Figure 11 is an exploded view of another example of the portion of the canister of Figure 3 having a support layer adjacent to a carrier layer that may be used with some embodiments;
Figure 12 is an exploded view of another example of the portion of the canister of Figure 3 having a support layer on both sides of an evaporative layer that may be used with some embodiments; and
Figure 13 is an exploded view of an example embodiment of a canister of the therapy system of Figure 1.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

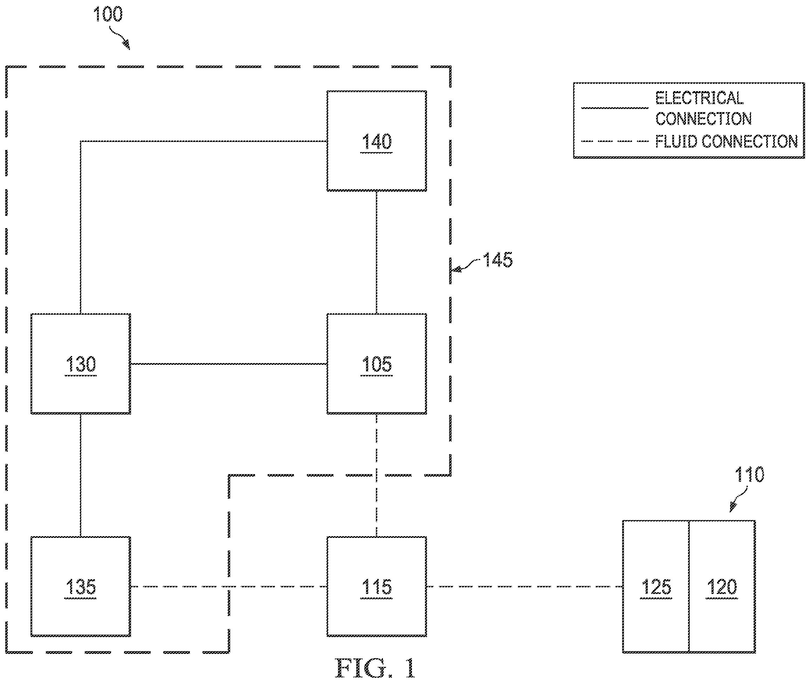

Figure 1 is a simplified functional block diagram of an example embodiment of a therapy system 100 that can provide negative-pressure therapy to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a canister 115, are examples of distribution components that may be associated with some examples of the therapy system 100. As illustrated in the example of Figure 1, the dressing 110 may comprise or consist essentially of a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130 and other components into a therapy unit 145.

In general, components of the therapy system 100 may be coupled directly or indirectly. For example, the negative-pressure source 105 may be directly coupled to the canister 115 and may be indirectly coupled to the dressing 110 through the canister 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The canister 115 is representative of a container, canister, pouch, or other storage component, which can be used to manage exudates and other fluids withdrawn from a tissue site. In many environments, a rigid canister may be preferred or required for collecting, storing, and disposing of fluids. In other environments, fluids may be properly disposed of without rigid canister storage, and a re-usable canister could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 120 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) (1 inch = 2.54 cm) may be particularly suitable for some types of therapy. The tensile strength of the tissue interface 120 may also vary according to needs of a prescribed therapy. The 25% compression load deflection of the tissue interface 120 may be at least 0.35 pounds (1 pound = 0.454 kg) per square inch, and the 65% compression load deflection may be at least 0.43 pounds per square inch. In some embodiments, the tensile strength of the tissue interface 120 may be at least 10 pounds per square inch. The tissue interface 120 may have a tear strength of at least 2.5 pounds per inch. In some embodiments, the tissue interface may be foam comprised of polyols such as polyester or polyether, isocyanate such as toluene diisocyanate, and polymerization modifiers such as amines and tin compounds. In some examples, the tissue interface 120 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

The tissue interface 120 may be either hydrophobic or hydrophilic. In an example in which the tissue interface 120 may be hydrophilic, the tissue interface 120 may also wick fluid away from a tissue site, while continuing to distribute negative pressure to the tissue site. The wicking properties of the tissue interface 120 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 120 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 120 may further serve as a scaffold for new cell-growth, or a scaffold material may be used in conjunction with the tissue interface 120 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and this descriptive convention should not be construed as a limiting convention.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in canister 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

Negative-pressure therapy has been repeatedly shown to be effective in the treatment of difficult to heal wounds. Manufacturers are designing current negative-pressure therapy systems to include evaporative canisters that are designed to process more liquids than the canister can physically retain at one time. Current structure and manufacturing processes for these canisters have resulted in leakage of the fluids stored inside the canisters. Negative-pressure therapy systems having improved canister structures and improved manufacturing processes for negative-pressure therapy systems may provide a long sought but unsolved solution in the art.

The canister 115 of the therapy system 100 may address these and other problems by providing a canister having an improved manufacturing process. The canister 115 may also include features that enable evaporation of liquids received inside the canister 115. By enabling evaporation, the canister 115 may process more liquids than the canister 115 can physically retain during a given time period than other canisters. The figures may illustrate exemplary embodiments of the canister 115; however, other exemplary canisters may have other sizes, shapes, and/or configurations that fall within the scope of the described and illustrated embodiments.

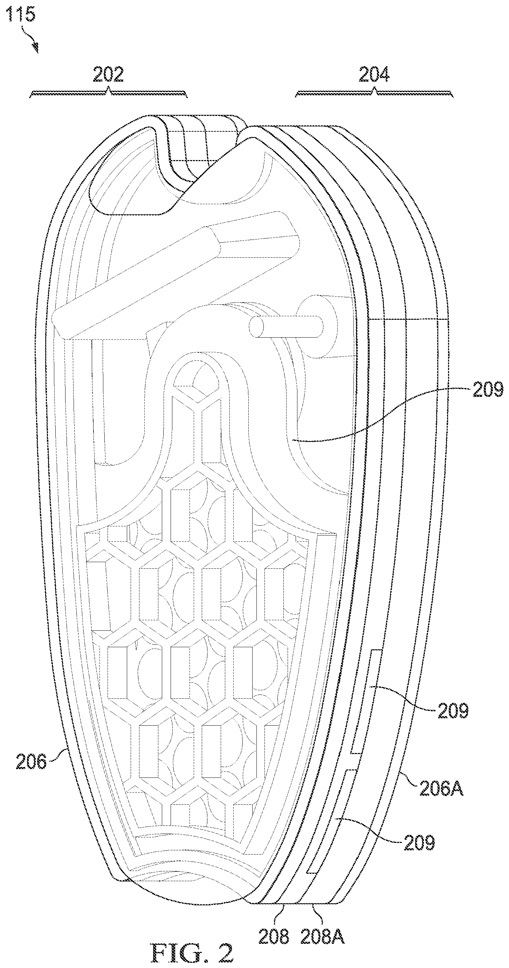

Figure 2 is an assembled view of the canister 115 illustrating additional details that may be associated with some embodiments of the therapy system 100. The canister 115 can include a first section, such as a first canister section 202 and a second section such as a second canister section 204. The first canister section 202 may have a first outer section 206 and a first filter carrier 208, and the second canister section 204 may have a second outer section 206A and a second filter carrier 208A. The first canister section 202 can be coupled to the second canister section 204 to create a chamber that is sealed from the ambient environment. The chamber can be formed by coupling the first outer section 206, to the first filter carrier 208 to form the first canister section 202 and coupling the second outer section 206A to the second filter carrier 208A to form the second canister section 204. The first canister section 202 may be fluidly connected to a dressing, such as the dressing 110. The second canister section 204 may be configured to be fluidly connected to a negative-pressure source, such as the negative-pressure source 105. The canister 115 comprises a pathway such as a fluid flow pathway 209. The fluid flow pathway 209 may be disposed between the first canister section 202 and the second canister section 204. The fluid flow pathway 209 is configured to allow transmission of evaporated fluids from the canister 115 to the ambient environment.

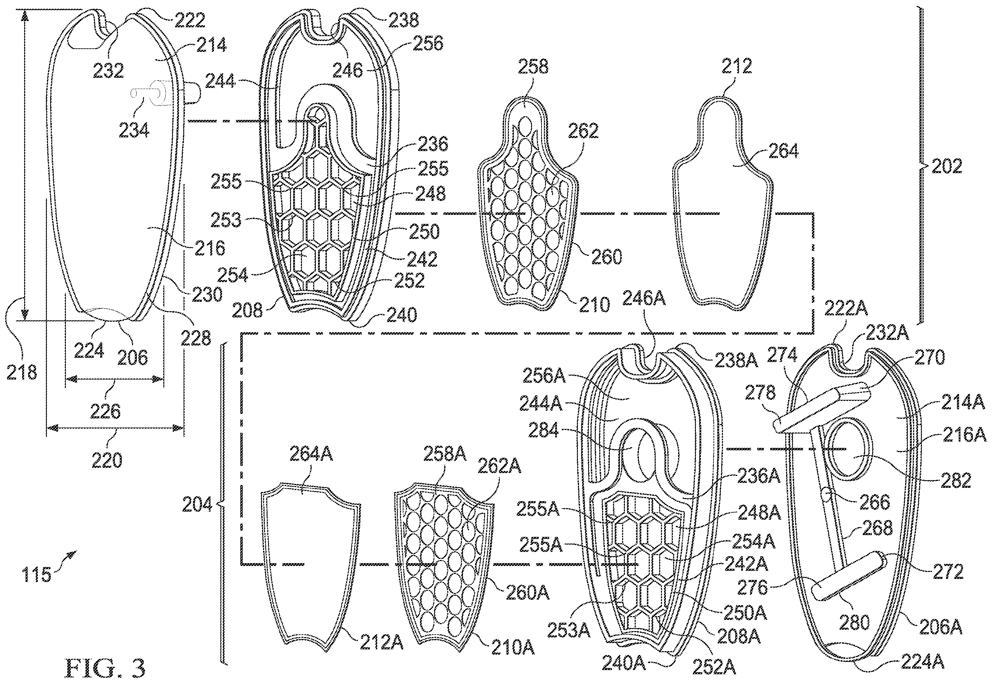

Figure 3 is an exploded view illustrating details that may be associated with some embodiments of the canister 115 of the therapy system 100. In some embodiments, the first canister section 202 and the second canister section 204 may both be configured to store fluid.

The first canister section 202 may comprise an outer wall, an exterior wall, a first wall, a first outer wall, or a first outer section 206, a carrier, a first carrier, or a first filter carrier 208, a perforated layer or a first support layer 210, and an evaporative layer or a first evaporative layer 212. In some embodiments, the first outer section 206 may have a base 214 with a first side or a first surface 216 and a second side or a second surface opposite the first surface 216. The first surface 216 may have a generally convex shape and may face away from the second canister section 204. The second surface may be concave shaped and may face the second canister section 204. In an illustrative embodiment, the base 214 may have a generally ovular shape having a flattened end. In other embodiments, the base 214 may be rectangular, circular, triangular, ovular, or amorphous in shape. The base 214 may have a length 218, and a width 220 at a first end 222, the width 220 may be perpendicular to the length 218. There may be a second end 224 opposite the first end 222. The second end 224 may have a width 226. In some embodiments, the width 220 may be greater than the width 226. In other embodiments, the width 220 may be substantially equal to the width 226. In some embodiments, the length 218 may be greater than the width 220 and the width 226. In other embodiments, the length 218 may be substantially equal to the width 220 and the width 226.

The base 214 may have a periphery or exterior edge 228. An extension 230 may extend from the exterior edge 228 of the base 214 towards the second canister section 204. The extension 230 may be coincident with the exterior edge 228 of the base 214. The extension 230 may extend from and be perpendicular to a plane defined by the exterior edge 228 of the base 214. In some embodiments, the exterior edge 228 may be a joint of the extension 230 and the base 214. For example, the exterior edge 228 may be formed by coupling the extension 230 to the base 214. In some embodiment, the exterior edge 228 may comprise a chamfered or beveled edge. In other embodiments, the exterior edge 228 may comprise a vertex of a perpendicular angle between the extension 230 and the base 214. In some embodiments, a receiver or a notch 232 can be formed in the first end 222. In some embodiments, the notch 232 extends through the base 214 from the first surface 216 to the second surface. The notch 232 may be positioned proximate a center of the width 220. In other embodiments, the notch 232 may not be centered on the width 220. In some embodiments, the notch 232 may have a width less than the width 220.

A fluid inlet 234 can be disposed in the first outer section 206. In some embodiments, the fluid inlet 234 may provide fluid communication across the base 214. In some embodiments, the fluid inlet 234 may be configured to fluidly couple the canister 115 to the dressing 110. For example, the fluid inlet 234 may be a fluid port configured to receive a fluid conductor such as a tube having at least one lumen. The fluid conductor may be coupled to the fluid inlet 234 and similarly coupled to the dressing 110. In this manner, the canister 115 may be fluidly coupled to the dressing 110. In some embodiments, the fluid inlet 234 may be disposed proximate the first end 222 of the base 214. In some embodiments, the fluid inlet 234 may be positioned near a midpoint of the width 220 at the first end 222. In the illustrative embodiment, the fluid inlet 234 may be separated from a center of the width 220 of the first end 222. In other embodiments, the fluid inlet 234 may be disposed in other locations on the base 214.

The first filter carrier 208 may comprise a first side or a first surface 236 and a second side or a second surface opposite the first surface 236. The first filter carrier 208 may have a first end 238 and a second end 240 opposite the first end 238. The first end 238 may have a width substantially equal to the width 220 of the first outer section 206, and the second end 240 may have a width substantially equal to the width 226 of the first outer section 206. In other embodiments, the first end 238 may have a width less than the width 220, and the second end 240 may have a width less than the width 226. The first filter carrier 208 may have a length that is substantially equal to the length 218 of the first outer section 206. In still other embodiments, the first filter carrier 208 may comprise a different size or shape.

The first surface 236 of the first filter carrier 208 may be facing the first outer section 206 and the second surface of the first filter carrier 208 may be facing the first support layer 210. The first filter carrier 208 may comprise a first portion 242 and a second portion 244. The first portion 242 may extend from the second end 240 to the second portion 244. The second portion 244 may extend from the first portion 242 to the first end 238.

In some embodiments, a receiver or a notch 246 can be formed in the first end 238. In some embodiments, the notch 246 extends through the first filter carrier 208 from the first surface 236 to the second surface. The notch 246 may be positioned proximate to a center of the width of the first end 238. In other embodiments, the notch 246 may not be centered on the width of the first end 238. In some embodiments, the notch 246 may have a width less than the width of the first end 238. In some embodiments, the notch 246 may be aligned or coincident with the notch 232 of the first outer section 206.

The first portion 242 may have a periphery or a peripheral portion, such as a first filter boundary 250 surrounding and defining an opening 248. In some embodiments, the opening 248 can comprise a substantial portion of the first portion 242. For example, the opening 248 may comprise greater than 50% of the surface area of the first portion 242. In some embodiments, the opening 248 may comprise about 50% to 70% of the surface area of the first portion 242. In other embodiments, the opening 248 may be about 90% or greater of the surface area of the first portion 242. A first support framework, such as a first filter section 252 can be disposed in the opening 248.

The first filter section 252 can comprise a plurality of arms, beams, or braces 253 extending across the opening 248. In some embodiments, the first filter section 252 forms a plurality of holes 254. Each hole 254 of the plurality of holes 254 may have a hexagonal shape. In some embodiments, each vertex 255 of each hole 254 may be proximate to at least one vertex 255 of an adjacent hole 254. In other embodiments, the plurality of holes 254 may comprise different sizes and shapes. The plurality of holes 254 may be formed in the first portion 242 by cutting, tearing, melting, forming, molding, or any other suitable method of creating a hole or opening. The plurality of holes 254 may maintain all or substantially all of the opening 248 of the first portion 242. The plurality of holes 254 of the first filter section 252 may be configured to allow fluid flow across the first filter section 252.

An opening 256 can be disposed in the second portion 244. The opening 256 may be formed in the second portion 244 of the first filter carrier 208 by cutting, tearing, melting, forming, molding, or any other suitable method of creating a hole or opening. The opening 256 can be configured to provide fluid communication across the first filter carrier 208 through the second portion 244. In some embodiments, the opening 256 can comprise a substantial portion of the second portion 244. For example, the opening 256 may comprise greater than 50% of the surface area of the second portion 244. In other embodiments, the opening 256 may be about 90% or greater of the surface area of the second portion 244.

The first filter carrier 208 may be coupled to the first outer section 206. For example, the periphery of the first surface 236 of the first filter carrier 208 may be coupled to the extension 230 of the first outer section 206. In some embodiments, the first filter carrier 208 may be coupled to the first outer section 206 at one or more attachment points. In other embodiments, the first filter carrier 208 and the first outer section 206 may be coupled by compression gaskets, double sided adhesives, a weld, or any other suitable method of coupling to seal the first filter carrier 208 to the first outer section 206.

The first support layer 210 has a first side or a first surface 258 and a second side or a second surface opposite the first surface 258. The first support layer 210 may be substantially the same shape and as the first portion 242 of the first filter carrier 208. In other embodiments, the first support layer 210 may be differently shaped. The first support layer 210 may be smaller in size than the first portion 242 of the first filter carrier 208 such that a periphery 260 of the first support layer 210 aligns with the first filter boundary 250 of the first filter carrier 208. The first surface 258 of the first support layer 210 may be configured to cover the opening 248 of the first filter carrier 208. The first surface 258 of the first support layer 210 may be coupled to the second surface of the first filter carrier 208 along the first filter boundary 250.

The first support layer 210 may be perforated with a plurality of holes 262. The plurality of holes 262 may be formed in the first support layer 210 by cutting, tearing, melting, forming, molding, or any other suitable method of creating a hole or opening. Each hole 262 of the plurality of holes 262 may have a circular shape. In other embodiments, the plurality of holes 262 may comprise different sizes and shapes. The plurality of holes 262 may maintain all or substantially all of the surface area of the first support layer 210. The plurality of holes 262 of the first support layer 210 may be configured to allow fluid flow across the first support layer 210. In some embodiments, each hole 262 of the plurality of holes 262 may be aligned with a corresponding hole 254 of the plurality of holes 254. In other embodiments, the holes 262 may be misaligned from the plurality of holes 254.

The first evaporative layer 212 comprises a first side or a first surface 264 and a second side or a second surface opposite the first surface 264. In some embodiments, the first evaporative layer 212 may comprise one or more evaporative membrane layers, for example, two evaporative membrane layers coupled to each other. The first evaporative layer 212 may be a continuous sheet having substantially the same size and shape as the first support layer 210. The first surface 264 of the first evaporative layer 212 may be configured to couple to the second surface of the first support layer 210. In some embodiments, the first support layer 210 and the first evaporative layer 212 may be welded to the first filter carrier 208 along the first filter boundary 250. In some preferred embodiments, the first support layer 210 and the first evaporative layer 212 may be welded to the first filter carrier 208 with an ultrasonic weld. In other embodiments, the first support layer 210, the first evaporative layer 212, and the first filter carrier 208 may be coupled by other methods such as adhesives, compression gaskets, or other attachment methods.

The second canister section 204 may comprise an outer wall, an exterior wall, a second wall, a second outer wall, or a second outer section 206A, a carrier, a second carrier, or a second filter carrier 208A, a perforated layer or a second support layer 210A, and an evaporative layer or a second evaporative layer 212A. In some embodiments, the second outer section 206A may have a substantially ovular base 214A. In other embodiments, the base 214A may be circular, triangular, rectangular, or amorphous in shape. The base 214A may have a first side or a first surface 216A and a second side or a second surface opposite the first surface 216A. The base 214A may have a length substantially equal to length 218 of the first outer section 206. The base may have a first end 222A with a width substantially equal to width 220 and a second end 224A opposite the first end 222A with a width substantially equal to width 226. In some embodiments, the width of the first end 222A may be greater than the width of the second end 224A. In other embodiments, the width of the first end 222A may be substantially equal to the width of the second end 224A. In some embodiments, the length may be greater than the width of the first end 222A and the width of the second end 224A. In other embodiments, the length may be substantially equal to the width of the first end 222A and the width of the second end 224A.

In some embodiments, a receiver or a notch 232A may be formed in the first end 222A. In some embodiments, the notch 232A extends through the base 214A from the first surface 216A to the second surface. The notch 232A may be positioned proximate to a center of the width of the first end 222A. In other embodiments, the notch 232A may not be centered on the width of the first end 222A. In some embodiments, the notch 232A may have a width less than the width of the first end 222A.

A negative-pressure inlet 266 can be disposed in the second outer section 206A. The negative-pressure inlet 266 may be substantially centered with respect to the length but may be off-centered with respect to the width of the second outer section 206A. In other embodiments, the negative-pressure inlet 266 may be substantially centered with respect to both the length and the width or may be located in another area in the second outer section 206A. In some embodiments, the negative-pressure inlet 266 may be configured to fluidly couple the canister 115 to the negative-pressure source 105. For example, a fluid conductor may be coupled to the negative-pressure inlet 266 and similarly coupled to the negative-pressure source 105 to fluidly couple the canister 115 to the negative-pressure source 105. The negative-pressure inlet 266 may be fluidly coupled to a channel 268 disposed on the first surface 216A of the second outer section 206A. The channel 268 may extend from the negative-pressure inlet 266 towards the first end 222A and towards the second end 224A. The channel 268 may be fluidly coupled to at least one chamber. In some embodiments, the channel 268 may be fluidly coupled to a first chamber 270 and a second chamber 272. The first chamber 270 may be located proximate to the first end 222A. In some embodiments, the second chamber 272 may be proximate to the second end 224A. The first chamber 270 may extend through the canister 115 from the second canister section 204 to the first canister section 202. More specifically, the first chamber 270 may extend through an opening 256A in a second portion 244A of the second filter carrier 208A and the opening 256 in the second portion 244 of the first filter carrier 208. The second chamber 272 may be disposed in the second canister section 204 and may extend from the second outer section 206A towards the second filter carrier 208A.

The first chamber 270 may have an opening disposed on an end 274 of the first chamber 270 opposite the second outer section 206A. The second chamber 272 may have an opening disposed on an end 276 of the second chamber 272 opposite the second outer section 206A. The opening on end 274 may be covered by a hydrophobic filter 278 and the opening on end 276 may be covered by a hydrophobic filter 280. The hydrophobic filter 278 may prevent ingress of liquids from the canister 115 into the first chamber 270 and the hydrophobic filter 280 may prevent ingress of liquids from the canister 115 into the second chamber 272. The volume of the first chamber 270 may be greater than the volume of the second chamber 272. In some embodiments, the plane associated with the end 274 of the first chamber 270 may be substantially parallel to the plane associated with the end 276 of the second chamber 272. The plane associated with the end 274 and the plane associated with the end 276 may be substantially parallel with the base 214A of the second outer section 206A. In other embodiments, the end 274 of the first chamber 270 and the end 276 of the second chamber 272 may be oriented such that the plane associated with the end 274 of the first chamber 270 may not be parallel to the plane associated with the end 276 of the second chamber 272. The first chamber 270 and second chamber 272 allow the passage of reduced pressure between the negative-pressure source 105 and the canister 115. In other embodiments, the canister 115 may not have a channel 268, the first chamber 270, or the second chamber 272. The negative-pressure inlet 266 may be covered by a hydrophobic filter on the first surface 216A of the second outer section 206A of the canister 115.

The second outer section 206A may further comprise a therapy unit connection 282 disposed in the second outer section 206A. The therapy unit connection 282 may be configured to fluidly couple the canister 115 to the therapy unit 145. The therapy unit connection 282 may be configured to allow a fluid flow from the therapy unit 145 to reach the fluid flow pathway 209 disposed between the first canister section 202 and the second canister section 204. The therapy unit connection 282 may be substantially circular. In other embodiments, the therapy unit connection 282 may be rectangular, ovular, triangular, or another shape. The therapy unit connection 282 may be substantially centered with respect to the width of the base 214A of the second outer section 206A but may be closer to the first end 222A than the second end 224A of the base 214A of the second outer section 206A. In other embodiments, the therapy unit connection 282 may be at a different location of the base 214A but may still fluidly couple the second outer section 206A to the therapy unit 145.

The second filter carrier 208A comprises a first side or a first surface 236A and a second side or a second surface opposite the first surface 236A. The second filter carrier 208A may have a first end 238A and a second end 240A opposite the first end 238A. The first end 238A may have a width substantially equal to the width 220 of the first outer section 206 and the second end 240A may have a width substantially equal to the width 226 of the first outer section 206. In other embodiments, the first end 238A may have a width less than the width 220 and the second end 240A may have a width less than the width 226. The second filter carrier 208A may have a length that is substantially equal to the length 218 of the first outer section 206. In other embodiments, the second filter carrier 208A may comprise a different size or shape.

The first surface 236A of the second filter carrier 208A may be facing the second support layer 210A and the second surface of the second filter carrier 208A may be facing the second outer section 206A. The second filter carrier 208A may comprise a first portion 242A and a second portion 244A. The first portion 242A may extend from the second end 240A to the second portion 244A. The second portion 244A may extend from the first portion 242A to the first end 238A.

In some embodiments, a receiver or a notch 246A can be formed in the first end 238A. In some embodiments, the notch 246A extends through the second filter carrier 208A from the first surface 236A to the second surface. The notch 246A may be positioned proximate to a center of the width of the first end 238A. In other embodiments, the notch 246A may not be centered on the width of the first end 238A. In some embodiments, the notch 246A may have a width less than the width of the first end 238A. In some embodiments, the notch 246A may be aligned with the notch 232A of the second outer section 206A.

The first portion 242A of the second filter carrier 208A may have a first opening 248A and a second opening 284. The second opening 284 may be created in the first portion 242A of the second filter carrier 208A by cutting, tearing, melting, forming, molding, or any other suitable method of creating a hole or opening. There may be a periphery or a peripheral portion, such as a second filter boundary 250A surrounding and defining the first opening 248A. For example, the first opening 248A may comprise greater than 50% of the surface area of the first portion 242A. In some embodiments, the opening 248 may comprise about 50% to 70% of the surface area of the first portion 242. In other embodiments, the first opening 248A may be about 90% or greater of the surface area of the first portion 242A. A first support framework, such as a second filter section 252A can be disposed in the first opening 248A.

The second filter section 252A can comprise a plurality of arms, beams, or braces 253A extending across the first opening 248A. In some embodiments, the second filter section 252A forms a plurality of holes 254A. Each hole 254A of the plurality of holes 254A may have a hexagonal shape. In some embodiments, each vertex 255A of each hole 254A may be proximate to at least one vertex 255A of an adjacent hole 254A. In other embodiments, the plurality of holes 254A may comprise different sizes and shapes. The plurality of holes 254A may be formed in the first portion 242A by cutting, tearing, melting, forming, molding, or any other suitable method of creating a hole or opening. The plurality of holes 254A may maintain all or substantially all of the first opening 248A of the first portion 242A. The plurality of holes 254A of the second filter section 252A may be configured to allow fluid flow across the second filter section 252A.

The second opening 284 of the first portion 242A may be disposed between the first opening 248A and the second portion 244A of the second filter carrier 208A. The second opening 284 may be substantially centered along the width of the second filter carrier 208A. The second opening 284 may be configured to extend through the second filter carrier 208A and couple to the therapy unit connection 282 of the second outer section 206A. The second opening 284 may provide a sealed chamber between the second filter carrier 208A and the second outer section 206A for a fluid flow from the therapy unit 145 to reach the fluid flow pathway 209 disposed between the first canister section 202 and the second canister section 204.

An opening 256A can be disposed in the second portion 244A. The opening 256A may be formed in the second portion 244A of the second filter carrier 208A by cutting, tearing, melting, forming, molding, or any other suitable method of creating a hole or opening. The opening 256A can be configured to provide fluid communication across the second filter carrier 208A through the second portion 244A. In some embodiments, the opening 256A can comprise a substantial portion of the second portion 244A. For example, the opening 256A may comprise greater than 50% of the surface area of the second portion 244A. In other embodiments, the opening 256A may be about 90% or greater of the surface area of the second portion 244A.

The second surface of the second filter carrier 208A may be coupled to the first surface 216A of the second outer section 206A. In some embodiments, the second filter carrier 208A may be coupled to the second outer section 206A at one or more attachment points. In other embodiments, the second filter carrier 208A and the second outer section 206A may be coupled by compression gaskets, double sided adhesives, a weld, or any other suitable method of coupling to seal the second filter carrier 208A to the second outer section 206A.

The second support layer 210A may have a first side or a first surface 258A and a second side or a second surface opposite the first surface 258A. The second support layer 210A may be substantially the same shape and as the first opening 248A of the first portion 242A of the second filter carrier 208A. In other embodiments, the second support layer 210A may be differently shaped. A periphery 260A of the second support layer 210A may align with the second filter boundary 250A of the second filter carrier 208A. The second surface of the second support layer 210A may be configured to cover the first opening 248A of the second filter carrier 208A. The second surface of the second support layer 210A may be coupled to the first surface 236A of the second filter carrier 208A along the second filter boundary 250A.

The second support layer 210A may be perforated with a plurality of holes 262A. The plurality of holes 262A may be formed in the second support layer 210A by cutting, tearing, melting, forming, molding, or any other suitable method of creating a hole or opening. Each hole 262A of the plurality of holes 262A may have a circular shape. In other embodiments, the plurality of holes 262A may comprise different sizes and shapes. The plurality of holes 262A may maintain all or substantially all of the surface area of the second support layer 210A. The plurality of holes 262A of the second support layer 210A may be configured to allow fluid flow across the second support layer 210A. In some embodiments, each hole 262A of the plurality of holes 262A may be aligned with a corresponding hole 254A of the plurality of holes 254A. In other embodiments, the holes 262A may be misaligned from the plurality of holes 254A.

The second evaporative layer 212A comprises a first side or a first surface 264A and a second side or a second surface opposite the first surface 264A. The second evaporative layer 212A may comprise one or more evaporative membrane layers. The second evaporative layer 212A may be a continuous sheet having substantially the same size and shape as the second support layer 210A. The second surface of the second evaporative layer 212A may be configured to cover the first surface 258A of the second support layer 210A. In some embodiments, the second support layer 210A and the second evaporative layer 212A may be welded to the second filter carrier 208A along the second filter boundary 250A. In some preferred embodiments, the second support layer 210A and the second evaporative layer 212A may be welded to the second filter carrier 208A with an ultrasonic weld. In other embodiments, the second support layer 210A, the second evaporative layer 212A, and the second filter carrier 208A may be coupled by other methods such as adhesives, compression gaskets, or other attachment methods.

When manufacturing the canister 115, the first outer section 206, the first filter carrier 208, the first support layer 210, and the first evaporative layer 212 are provided to create the first canister section 202. The first support layer 210 is coupled to the first filter carrier 208 such that the first surface 258 of the first support layer 210 is proximate to the second surface 404 of the first filter carrier 208. The first evaporative layer 212 is coupled to the first support layer 210 such that the first surface 264 of the first evaporative layer 212 is proximate to the second surface 414 of the first support layer 210. The first filter carrier 208, the first support layer 210, and the first evaporative layer 212 may be coupled to the first outer section 206 such that the first surface 236 of the first filter carrier 208 is proximate to the second surface of the first outer section 206. The first filter carrier 208, the first support layer 210, the first evaporative layer 212, and the first outer section 206 may be coupled by welding, using adhesives, compression gaskets, or other attachment methods.

The second outer section 206A, the second filter carrier 208A, the second support layer 210A, and the second evaporative layer 212A may be provided to create the second canister section 204. The second support layer 210A is coupled to the second filter carrier 208A such that the second surface of the second support layer 210A is proximate to the first surface 236A of the second filter carrier 208A. The second evaporative layer 212A is coupled to the second support layer 210A such that the second surface of the second evaporative layer is proximate to the first surface 258A of the second support layer 210A. The second filter carrier 208A, the second support layer 210A, and the second evaporative layer 212A may be coupled to the second outer section 206A such that the second surface of the second filter carrier 208A is proximate to the first surface 216A of the second outer section 206A. The second filter carrier 208A, the second support layer 210A, the second evaporative layer 212A, and the second outer section 206A may be coupled by welding, using adhesives, compression gaskets, or other attachment methods.

In some embodiments, providing the first filter carrier 208 may further include forming the plurality of holes 254 in the first portion 242 and forming the opening 256 in the second portion 244. Providing the second filter carrier 208A may further include forming the plurality of holes 254A in the first portion 242A and forming the opening 256A in the second portion 244A. The plurality of holes 254, the opening 256, the plurality of holes 254A, and the opening 256A may be formed by cutting, tearing, melting, forming, molding, or any other suitable method of creating a hole or opening.

In some embodiments, providing the first support layer 210 may further include forming the plurality of holes 262. Providing the second support layer 210A may further include forming the plurality of holes 262A. The plurality of holes 262 and the plurality of holes 262A may be formed by cutting, tearing, melting, forming, molding, or any other suitable method of creating a hole or opening.

The canister 115 may be formed by coupling the first canister section 202 to the second canister section 204 such that the first filter carrier 208, the first support layer 210, the first evaporative layer 212, the second evaporative layer 212A, the second support layer 210A, and the second filter carrier 208A are disposed between the first outer section 206 and the second outer section 206A. When the first canister section 202 is coupled to the second canister section 204, the fluid flow pathway 209 may be formed between the first canister section 202 and the second canister section 204. The fluid flow pathway 209 may be fluidly connected to the therapy unit 145 through the chamber created between the second opening 284 of the first portion 242A of the second filter carrier 208A and the therapy unit connection 282 of the second outer section 206A. The fluid flow pathway 209 may extend from the second opening 284 of the first portion 242A of the second filter carrier 208A to an end of the canister 115. The fluid flow pathway 209 may be about 2mm thick. The fluid flow pathway 209 may extend through the canister 115 and open to ambient environment at the end of the canister 115 to allow evaporated fluids from the canister 115 to escape to the ambient environment.

The first outer section 206, the first filter carrier 208, the second filter carrier 208A, and the second outer section 206A may comprise a type of material having sufficient rigidity and structural integrity to withstand the reduced pressure required for negative-pressure treatment and to contain fluid therein. In some embodiments, the first outer section 206, the first filter carrier 208, the second filter carrier 208A, and the second outer section 206A may comprise a clear rigid plastic such as ABS or TPU. Some exemplary materials of the first outer section 206, the first filter carrier 208, the second filter carrier 208A, and the second outer section 206A are plastics, polymers, thermoplastics, metals, metal alloys, composition material, fiber-type materials, and other similar materials. The plastics described herein may be a substance or structure capable of being shaped or molded with or without the application of heat, a high polymer, usually synthetic, combined with other ingredients such as curatives, fillers, reinforcing agents, plasticizers, etc. Plastics can be formed or molded under heat and pressure in its raw state and machined to high dimensional accuracy, trimmed and finished in its hardened state. The thermoplastic type can be resoftened to its original condition by heat. In addition, the plastics may mean engineered plastics such as those that are capable of sustaining high levels of stress and are machinable and dimensionally stable. Some exemplary plastics are nylon, acetyls, polycarbonates, ABS resins, PPO/styrene, ISOPLAST 2530, TURLUX HS 2822, and polybutylene terephthalate. The thermoplastics described herein may be high polymers that soften when exposed to heat and return to their original condition when cooled to room temperature.

The first evaporative layer 212, the first support layer 210, the second support layer 210A, and the second evaporative layer 212A may be comprised of a thermoplastic polyurethane (TPU) material. In some embodiments, the TPU material may be a high moisture vapor transmission rate (MVTR) film. The high MVTR film should have an MVTR of about 2000 to 5000 g/m²/24hrs in an upright cup test. The first evaporative layer 212 and the second evaporative layer 212A may be about 15µm -50µm thick. In some embodiments, the first evaporative layer 212 and the second evaporative layer 212A may each include two layers of high MVTR film that may each be about 20µm thick. In other embodiments, the first evaporative layer 212 and the second evaporative layer 212A may each comprise one layer of high MVRT film that may be about 40µm thick. The first support layer 210 and the second support layer 210A may be about 50µm -500µm thick. In some optimal embodiments, the first support layer 210 and the second support layer 210A may be about 250µm thick. In some embodiments, the high MVTR film may be BASF E1385A 12000. In other embodiments, the high MVTR film may be COVESTRO VPT 9121, or COVESTRO Platilon U 250µm.

In some embodiments, the elements of the first canister section 202 and the second canister section 204 may comprise coatings that are designed to improve transfer rates of the canister 115. These coatings may create localized fluid gradients that may enable increased evaporation through the first evaporative layer 212 and the second evaporative layer 212A. The coatings may be polar so as to attract water molecules. The coatings may include nitrogen, oxygen, or fluorine to enable hydrogen bonding which may quickly remove the hydrogen molecules from the canister 115 and distribute them to the first evaporative layer 212 and the second evaporative layer 212A and into the fluid flow pathway 209. In other embodiments, the coatings may include halogens such as chlorine and bromine. Other coatings may include metal compounds or polymers such as sodium, potassium, or calcium. In some embodiments, the coatings may be plasma coatings or may be a corona treatment designed to oxidize the elements of the first canister section 202 and the second canister section 204 that they are applied to.

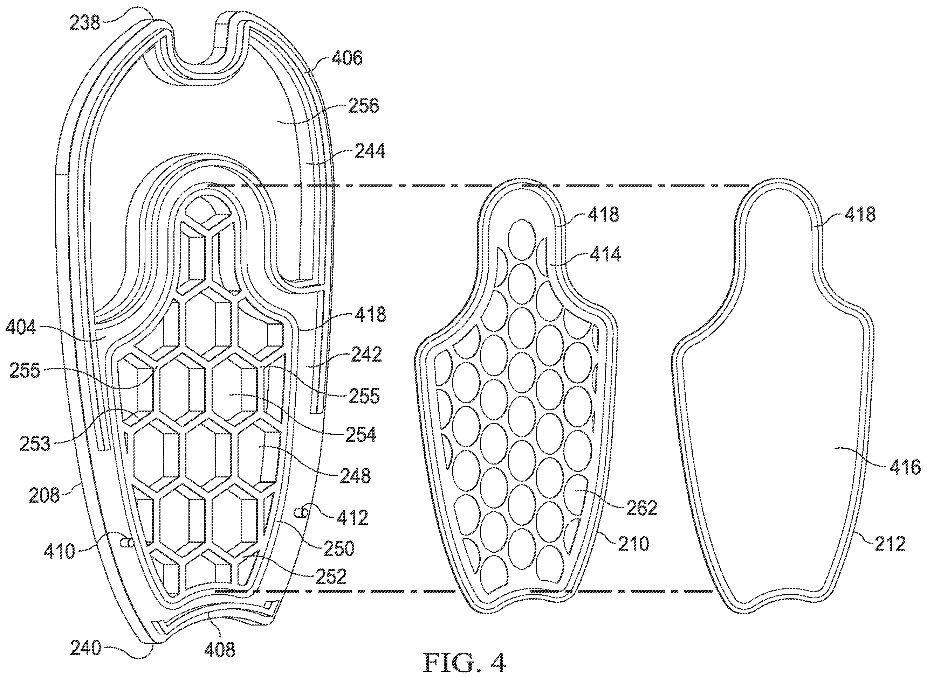

Figure 4 is an exploded view illustrating additional details that may be associated with some example embodiments of a portion of the first canister section 202. In Figure 4, the first filter carrier 208, the first support layer 210, and the first evaporative layer 212 are illustrated.

The first filter carrier 208 includes a second surface 404 opposite the first surface 236 of the first filter carrier 208. The second surface 404 faces the first support layer 210. The second surface 404 may include a first extension 406, a second extension 408, a third extension 410, and a fourth extension 412 extending away from the second surface 404 of the first filter carrier 208. In some embodiments, the first extension 406, the second extension 408, the third extension 410, and the fourth extension 412 may project towards the first support layer 210. The first extension 406 may surround the opening 256 of the second portion 244. For example, the first extension 406 may be an annular wall outboard of the opening 256 of the second portion 244. In some embodiments, at least a portion of the first extension 406 may further extend into the first portion 242 from the second portion 244. For example, the first extension 406 may include linear portions having a first end proximate the boundary between the first portion 242 and the second portion 244 and a second end proximate a midline of the first portion 242. The second extension 408 may be disposed proximate the second end 240. The second extension 408 may be inboard of the second end 240 and outboard of the first filter boundary 250 and the opening 248. The second extension may be substantially linear and have a length about equal to the width of the first filter carrier 208 along the second end 240. The third extension 410 and the fourth extension 412 may be cylindrical projections and protrude from the second surface 404 of the first filter carrier 208. The third extension 410 can be disposed along the periphery of the first portion 242. In some embodiments, the third extension 410 may be disposed about half way between the second end of the first extension 406 and the second extension 408. Similarly, the fourth extension 412 can be disposed along the periphery of the first portion 242. In some embodiments, the fourth extension 412 may be disposed about half way between the second end of the first extension 406 and the second extension 408. In some embodiments, the third extension 410 and the fourth extension 412 can be disposed on opposite sides of the opening 248.

The first extension 406, the second extension 408, the third extension 410, and the fourth extension 412 may couple the first filter carrier 208 to the second filter carrier 208A. The first extension 406, the second extension 408, the third extension 410, and the fourth extension 412 may have a height of about 2mm from the second surface 404 of the first filter carrier 208. In some embodiments, a gap formed between ends of the first extension 406, the second extension 408, the third extension 410, and the fourth extension 412 may create the fluid flow pathway 209 between the first canister section 202 and the second canister section 204. In other embodiments, the height of the first extension 406, the second extension 408, the third extension 410, and the fourth extension 412 may be greater than 2mm or less than 2mm from the second surface 404 of the first filter carrier 208. Preferably, a height of the first extension 406, the second extension 408, the third extension 410, and the fourth extension 412 may define a volume between the first evaporative layer 212 and the second evaporative layer 212A. In some embodiments, the fluid flow pathway 209 may be in fluid communication with the ambient environment via a separation between the first extension 406 and the third extension 410, the first extension 406 and the fourth extension 412, the third extension 410 and the second extension 408, and the fourth extension 412, and the second extension 408. The first extension 406, the second extension 408, the third extension 410, and the fourth extension 412 may be comprised of a similar material as the first filter carrier 208 having sufficient rigidity and structural integrity to withstand the application of a reduced pressure.

The first support layer 210 has a second surface 414 opposite the first surface 258. The first surface 258 of the first support layer 210 faces the second surface 404 of the first filter carrier 208. The second surface 414 of the first support layer 210 faces the first surface 264 of the first evaporative layer 212.

The first evaporative layer 212 has a second surface 416 opposite the first surface 264. The first surface 264 of the first evaporative layer 212 faces the second surface 414 of the first support layer 210. The second surface 416 of the first evaporative layer 212 may face the second canister section 204.

The first surface 258 of the first support layer 210 is coupled to the second surface 404 of the first filter carrier 208. The first surface 264 of the first evaporative layer 212 is coupled to the second surface 414 of the first support layer 210. A path such as connection path 418 may define where the first filter carrier 208, the first support layer 210, and the first evaporative layer 212 are coupled together. The connection path 418 may be located proximate to the first filter boundary 250 of the first filter carrier 208. The connection path 418 may be located proximate to the periphery of the first support layer 210 and the first evaporative layer 212.

In some embodiments, the first filter carrier 208, the first support layer 210, and the first evaporative layer 212 may be coupled together with a weld along the connection path 418. The weld may extend from the second surface 416 of the first evaporative layer 212, through the first support layer 210 and to the second surface 404 of the first filter carrier 208.

In other embodiments, the first filter carrier 208, the first support layer 210, and the first evaporative layer 212 may be coupled along the connection path 418 with double sided adhesive. A first double sided adhesive may be disposed on the first surface 264 of the first evaporative layer 212 along the connection path 418. The first double sided adhesive may couple the first surface 264 of the first evaporative layer 212 to the second surface 414 of the first support layer 210. A second double sided adhesive may be disposed on the first surface 258 of the first support layer 210 along the connection path 418. The second double sided adhesive may couple the first surface 258 of the first support layer 210 to the second surface 404 of the first filter carrier 208. In other embodiments, the first double sided adhesive may be disposed on the second surface 414 of the first support layer 210 along the connection path 418. The first double sided adhesive may couple the second surface 414 of the first support layer 210 to the first surface 264 of the first evaporative layer 212. The second double sided adhesive may be disposed on the second surface 404 of the first filter carrier 208 along the connection path 418. The second double sided adhesive may couple the second surface 404 of the first filter carrier 208 to the first surface 258 of the first support layer 210. In other embodiments, the first double sided adhesive may be disposed along the connection path 418 on the second surface 414 of the first support layer 210 and the second double sided adhesive may be disposed along the connection path 418 on the first surface 258 of the first support layer 210. In other embodiments, the first double sided adhesive may be disposed along the connection path 418 on the first surface 264 of the first evaporative layer 212 and the second double sided adhesive may be disposed along the connection path 418 on the second surface 404 of the first filter carrier 208.

In other embodiments, a compression gasket may be disposed on the second surface 414 of the first filter carrier 208 along the connection path 418. The compression gasket may be over molded into the first filter carrier 208. In other examples, the compression gasket may be a separate element that is coupled to the second surface 404 of the first filter carrier 208 along the connection path 418. In some embodiments, the compression gasket may be welded to the second surface 414 of the first filter carrier 208. The first support layer 210 and the first evaporative layer 212 may be coupled to the compression gasket such that the first filter carrier 208, the first support layer 210, and the first evaporative layer 212 are sealed to each other.

In some embodiments, fluid collected from a tissue site may flow through the holes 254 of the first filter carrier 208 and the holes 262 of the first support layer 210 into contact with the first evaporative layer 212. Fluids in contact with the first evaporative layer 212 may transfer through the first evaporative layer 212 by osmosis to reach the fluid flow pathway 209.

In some embodiments, a portion of the second canister section 204 including the second filter carrier 208A, the second support layer 210A, and the second evaporative layer 212A may be coupled together with a weld, an adhesive, or a compression gasket to create a fluid seal so that fluids cannot leak into the fluid flow pathway 209 from the second canister section 204. There may be a connection path located proximate to the second filter boundary 250A of the second filter carrier 208A. The connection path may also be located proximate to the periphery of the second support layer 210A and the second evaporative layer 212A. The second filter carrier 208A, the second support layer 210A, and the second evaporative layer 212A may be coupled together by any of the above described methods along the connection path.

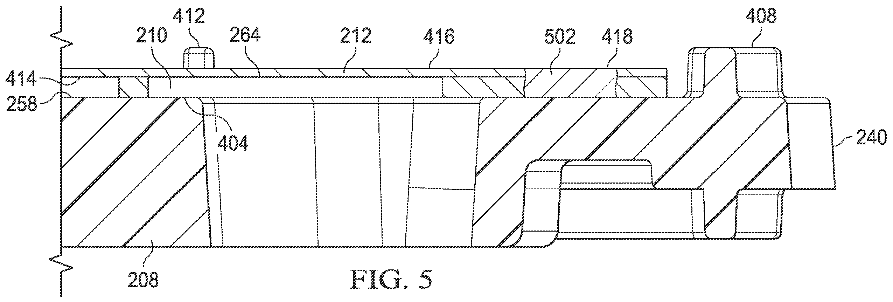

Figure 5 is a perspective view illustrating additional details that may be associated with the portion of the first canister section 202 of Figure 4. The first surface 258 of the first support layer 210 is adjacent to the second surface 404 of the first filter carrier 208 and the first surface 264 of the first evaporative layer 212 is adjacent to the second surface 414 of the first support layer 210. The fourth extension 412 and the second extension 408 may project from the second surface 404 of the first filter carrier 208. The fourth extension 412 and the second extension 408 may extend from the first filter carrier 208 past the first support layer 210 and the first evaporative layer 212. The fourth extension 412 and the second extension 408 may be configured to couple to the second filter carrier 208A such that the fluid flow pathway 209 may be formed between the first canister section 202 and the second canister section 204.

The first filter carrier 208, the first support layer 210, and the first evaporative layer 212 may be coupled together by a weld 502. The weld 502 may follow the connection path 418. The weld 502 may extend from the second surface 416 of the first evaporative layer 212, through the first support layer 210 and to the second surface 404 of the first filter carrier 208. In other embodiments, the first filter carrier 208, the first support layer 210, and the first evaporative layer 212 may be coupled by any of the methods described in detail above, such as adhering, bonding, friction coupling, etc.

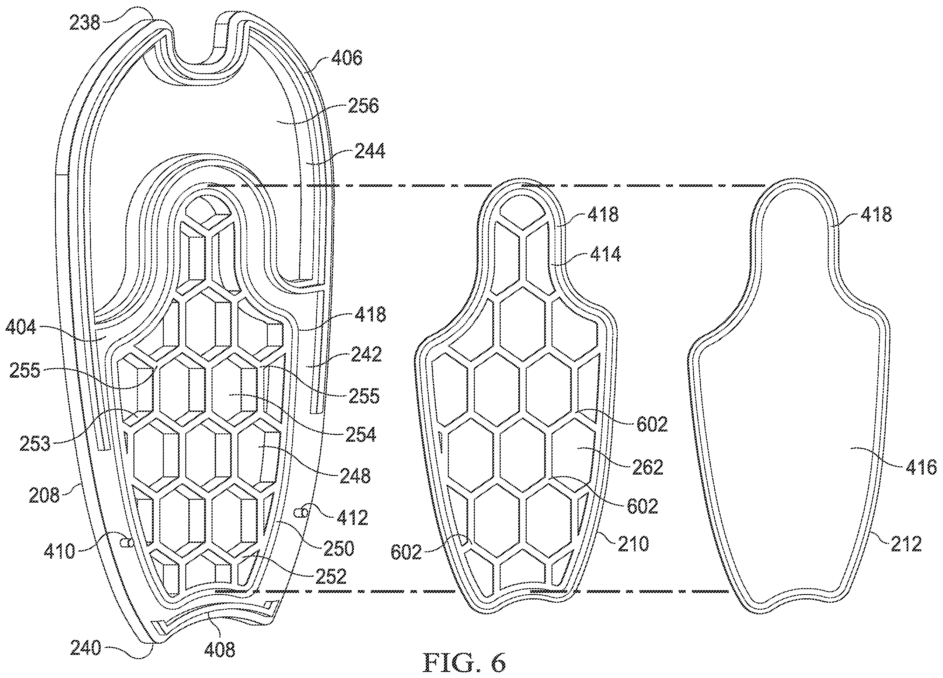

Figure 6 is an exploded view illustrating additional details that may be associated with some example embodiments of a portion of the first canister section 202. As described with respect to Figure 2 and Figure 3, the first canister section 202 may include the first outer section 206, the first filter carrier 208, the first support layer 210, and the first evaporative layer 212. In Figure 6, the first filter carrier 208, the first support layer 210, and the first evaporative layer 212 are illustrated. In the illustrated embodiment, each hole 262 of the plurality of holes 262 of the first support layer 210 may have a hexagonal shape. Each hole 262 may have six vertices 602. Each vertex 602 of each hole 262 may be proximate to at least one vertex 602 of an adjacent hole 262. In some embodiments, the plurality of holes 262 of the first filter carrier 208 may be aligned with the plurality of holes 254 of the first support layer 210. If the holes 262 of the first support layer 210 are aligned with the holes 254 of the first filter carrier 208 it may be easier for fluid from the first canister section 202 to reach the first evaporative layer 212 because the fluid may not contact the first surface 258 of the first support layer 210. This alignment of the first filter carrier 208 and the first support layer 210 may increase the moisture vapor transmission rate of the canister 115. This may allow increase an amount of liquid that may be evaporated from the first canister section 202 through the first evaporative layer 212. In other embodiments, the holes 262 and the holes 254 may be misaligned to control the quantity of fluid in contact with the first evaporative layer 212.

In some embodiments, each hole 262A of the plurality of holes 262A of the second support layer 210A may have a hexagonal shape. Each hole 262A may have six vertices. Each vertex of each hole 262A may be proximate to at least one vertex of an adjacent hole 262A. In some embodiments, the plurality of holes 262A of the second filter carrier 208A may be aligned with the plurality of holes 254A of the second support layer 210A. If the holes 262A of the second support layer 210A are aligned with the holes 254A of the second filter carrier 208A it may be easier for fluid from the second canister section 204 to reach the second evaporative layer 212A because the fluid should not contact the second surface of the second support layer 210A. This alignment of the second filter carrier 208A and the second support layer 210A may increase the moisture vapor transmission rate of the canister 115. This may allow increase an amount of liquid that may be evaporated from the second canister section 204 through the second evaporative layer 212A. In other embodiments, the holes 262A and the holes 254A may be misaligned to control the quantity of fluid in contact with the second evaporative layer 212A.

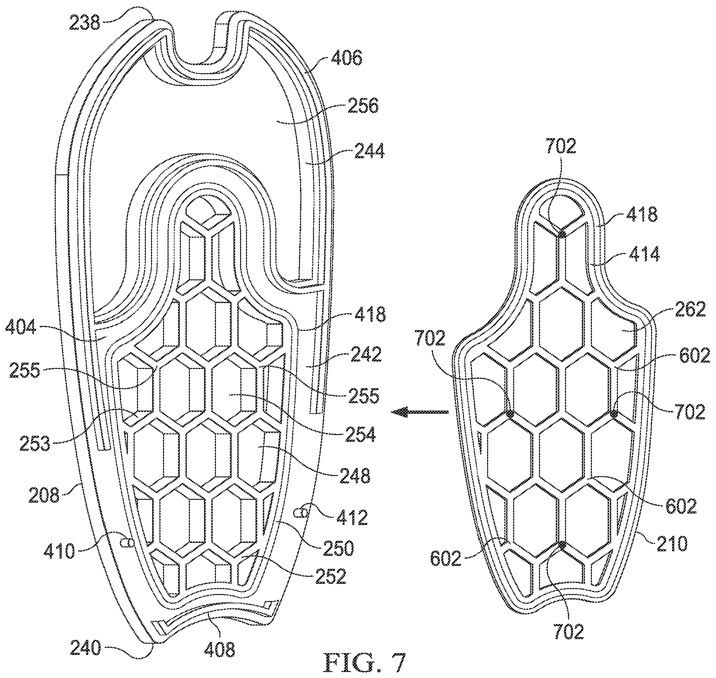

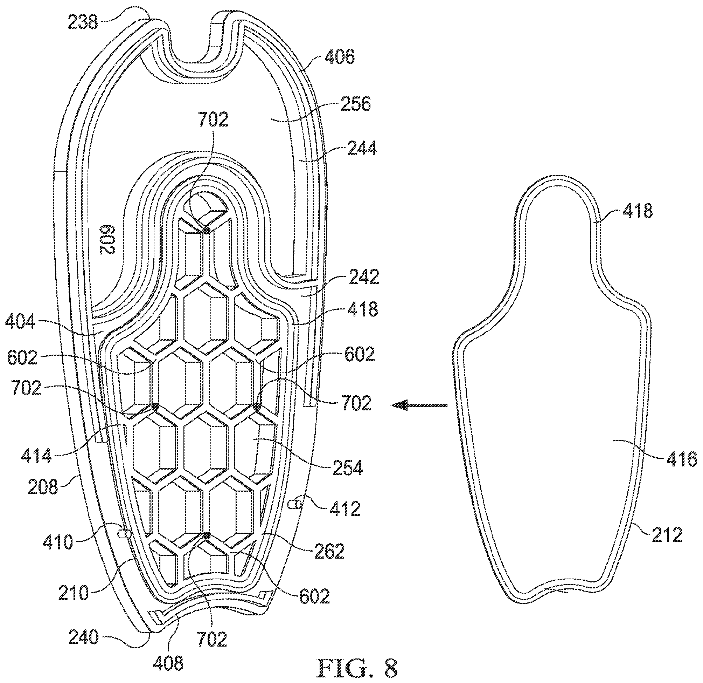

Figures 7 and 8 illustrate an assembly process coupling the first filter carrier 208, the first support layer 210, and the first evaporative layer 212 of Figure 6. The first step of the assembly process may be to align the plurality of holes 262 of the first support layer 210 with the plurality of holes 254 of the first filter carrier 208. In some embodiments, there may be a vacuum source located proximate to the first surface 236 of the first filter carrier 208. The vacuum source may be used to align the plurality of holes 262 of the first support layer 210 with the plurality of holes 254 of the first filter carrier 208. The next step, illustrated in Figure 7, may be to couple the first filter carrier 208 to the first support layer 210. In some embodiments, the first filter carrier 208 may be coupled to the first support layer 210 by at least one weld. In other embodiments, the first filter carrier 208 may be coupled to the first support layer 210 by another method such as adhering, bonding, friction coupling, the vacuum source, or another similar method.

In the illustrative embodiment, four vertices 602 of the first support layer 210 are coupled to four corresponding vertices 255 of the first filter carrier 208 with spot welds 702. The spot welds 702 may ensure that the plurality of holes 262 of the first support layer 210 and the plurality of holes 254 of the first filter carrier 208 are aligned prior to coupling the first evaporative layer 212 to the first support layer 210 and the first filter carrier 208 along the connection path 418. This alignment may optimize the amount of fluid that can reach the first evaporative layer 212 because fluid may not come into contact with the first surface 258 of the first support layer 210.

Figure 8 illustrates a final step coupling the first filter carrier 208, the first support layer 210, and the first evaporative layer 212 to form a portion of the first canister section 202. The first evaporative layer 212 is coupled to the first support layer 210 such that the first surface 264 of the first evaporative layer 212 is adjacent to the second surface 414 of the first support layer 210. The first evaporative layer 212 may be coupled to the first support layer 210 and the first filter carrier 208 along the connection path 418. In some embodiments, the first evaporative layer 212 may be coupled to the first support layer 210 with a weld along the connection path 418. In other embodiments, the first evaporative layer 212 may be coupled to the first support layer 210 by other methods such as adhering, bonding, friction coupling, compression gaskets or another method designed to ensure that liquids stored inside the canister 115 remain separate from the fluid flow pathway 209.

In some embodiments, a first step of an assembly process of coupling the second filter carrier 208A, the second support layer 210A, and the second evaporative layer 212A may be to align the plurality of holes 262A of the second support layer 210A with the plurality of holes 254A of the second filter carrier 208A. A second step may be to couple the second filter carrier 208A to the second support layer 210A. The second filter carrier 208A and the second support layer 210A may be coupled by at least one weld. For example, at least one vertex of the second support layer 210A may be coupled to the corresponding at least one vertex of the second filter carrier 208A with spot welds. The spot welds may ensure that the plurality of holes 262A of the second support layer 210A and the plurality of holes 254A of the second filter carrier 208A are aligned prior to coupling the second evaporative layer 212A to the second support layer 210A and the second filter carrier 208A along the connection path. This alignment may optimize the amount of fluid that can reach the second evaporative layer 212A because fluid may not come into contact with the second surface of the second support layer 210A. A third step of coupling the second filter carrier 208A, the second support layer 210A, and the second evaporative layer 212A may be to couple the second filter carrier 208A, the second support layer 210A, and the second evaporative layer 212A along the connection path. In some embodiments, the second evaporative layer 212A may be coupled to the second support layer 210A with a weld along the connection path. In other embodiments, the second evaporative layer 212A may be coupled to the second support layer 210A by other methods such as adhering, bonding, friction coupling, compression gaskets or another method designed to ensure that liquids stored inside the canister 115 remain separate from the fluid flow pathway 209.

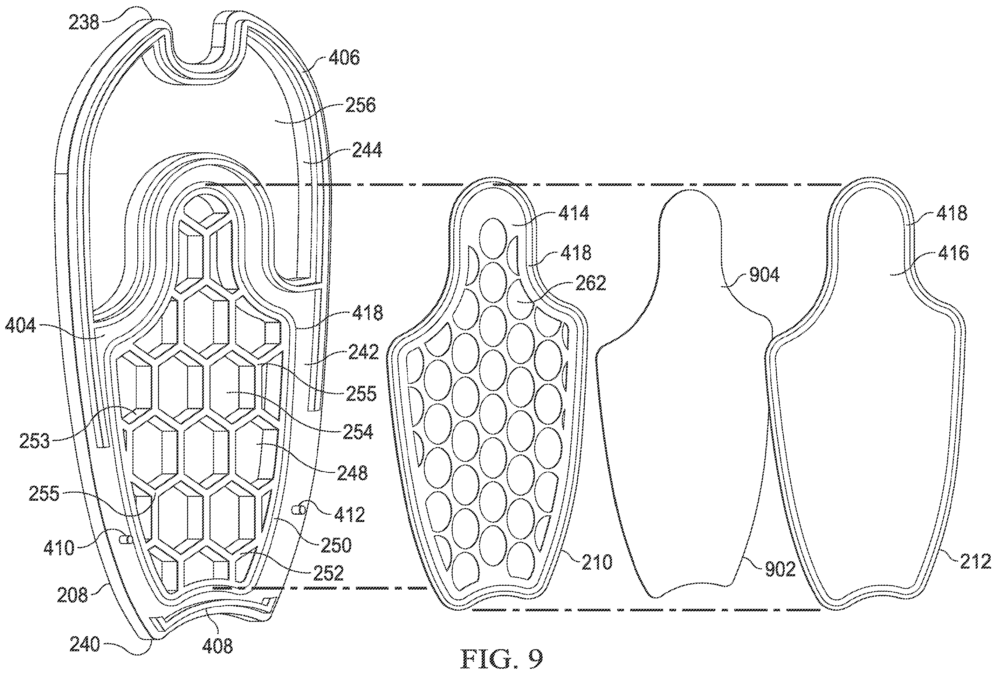

Figure 9 is an exploded view illustrating additional details that may be associated with some example embodiments of a portion of the first canister section 202. As described with respect to Figure 2 and Figure 3, the first canister section 202 may include the first outer section 206, the first filter carrier 208, the first support layer 210, and the first evaporative layer 212. In the embodiment illustrated in Figure 6, the first canister section 202 may further include a distribution layer, a manifold, a nonwoven, or a first nonwoven layer 902. The first nonwoven layer 902 may be disposed between the first support layer 210 and the first evaporative layer 212. In some embodiments, the first nonwoven layer 902 may have a first surface and a second surface 904 opposite the first surface. The first surface of the first nonwoven layer 902 may face the second surface 414 of the first support layer 210, and the second surface 904 of the first nonwoven layer 902 may face the first surface 264 of the first evaporative layer 212.

The first nonwoven layer 902 may have a shape similar to the first support layer 210 and the first evaporative layer 212. In some embodiments, the first nonwoven layer 902 may have a length and a width that are less than a length and a width of the first support layer 210 and the first evaporative layer 212. If the first support layer 210 is coupled to the first evaporative layer 212, the first nonwoven layer 902 may be located inboard of the connection path 418. If the first filter carrier 208, the first support layer 210, and the first evaporative layer 212 are coupled to each other along the connection path 418, the first nonwoven layer 902 may be excluded from the connection path 418. For example, if the first filter carrier 208, the first support layer 210, and the first evaporative layer 212 are welded together along the connection path 418, the first nonwoven layer 902 would be located inboard of the weld. Excluding the first nonwoven layer 902 from the connection path 418 may help to increase the stability of the first canister section 202. In some embodiments, the first nonwoven layer 902 may be spot welded to the first support layer 210 at at least one point. The at least one sport weld may hold the first nonwoven layer 902 in place, preventing interference with the connection path 418. This technique may increase the likelihood that the first nonwoven layer 902 is in a more optimal position between the first support layer 210 and the first evaporative layer 212.

The first nonwoven layer 902 may enable fluids from the first canister section 202 to be distributed across a majority of the surface area of the first evaporative layer 212. In embodiments including the first nonwoven layer 902, fluids may flow through the holes 262 of the first support layer 210 and the holes 254 of the first filter carrier 208 to reach the first nonwoven layer 902. Once in contact with the first nonwoven layer 902, fluids may be distributed across the first nonwoven layer 902 and may contact a maximum amount of the surface area of the first evaporative layer 212. If more fluids are able to contact the first evaporative layer 212, there may be an increase in the moisture vapor transmission rate of the canister 115.

The first nonwoven layer 902 may comprise material of grade BK091620-11 having a weight of about 158gsm. In other embodiments, the first nonwoven layer 902 may comprise Libeltex TDL2 or a similar material that may allow fluids from the canister 115 to reach first evaporative layer 212.. In some embodiments, the first nonwoven layer 902 may contain coatings or additives that help to distribute fluid and increase fluid contact with the first evaporative layer 212. In some embodiments, the first nonwoven layer 902 may contain a coating or additive that may help to distribute fluid and increase fluid contact with the first evaporative layer 212. The coating may be polar so as to attract water molecules. The coating may include nitrogen, oxygen, or flourine to enable hydrogen bonding which may quickly remove the hydrogen molecules from the canister 115 and distribute them to the first evaporative layer 212. In other embodiments, the coating may include halogens such as chlorine and bromine. In still other embodiments, the coating may include metal compounds or polymers such as sodium, potassium, or calcium. In some embodiments, the coating may be plasma coatings or may be a corona treatment designed to oxidize the first nonwoven layer 902 to provide a polar coating. The coating may be applied to the first surface of the first nonwoven layer 902. In other embodiments, the coatings may be applied to the first surface and the second surface 904 of the first nonwoven layer 902.

In some embodiments, the second canister section 204 may include a distribution layer, a manifold, a nonwoven, or a second nonwoven layer disposed between the second evaporative layer 212A and the second support layer 210A. The second nonwoven layer may have a first surface and a second surface opposite the first surface. The first surface of the second nonwoven layer may face the second surface of the second evaporative layer 212A, and the second surface of the second nonwoven layer may face the first surface of the second support layer 210A.

The second nonwoven layer may have a shape similar to the second support layer 210A and the second evaporative layer 212A. In some embodiments, the second nonwoven layer may have a length and a width that are less than a length and a width of the second support layer 210A and the second evaporative layer 212A. If the second support layer 210A is coupled to the second evaporative layer 212A, the second nonwoven layer may be located inboard of the connection path 418. If the second filter carrier 208A, the second support layer 210A, and the second evaporative layer 212A are coupled to each other along the connection path, the second nonwoven layer may be excluded from the connection path. For example, if the second filter carrier 208A, the second support layer 210A, and the second evaporative layer 212A are welded together along the connection path, the second nonwoven layer would be located inboard of the weld. Excluding the second nonwoven layer from the connection path may help to increase the stability of the second canister section 204. In some embodiments, the second nonwoven layer may be spot welded to the second support layer 210A at at least one point. The at least one sport weld may hold the second nonwoven layer in place, preventing interference with the connection path. This technique may increase the likelihood that the second nonwoven layer is in a more optimal position between the second support layer 210A and the second evaporative layer 212A.

The second nonwoven layer may enable fluids from the second canister section 204 to be distributed across a majority of the surface area of the second evaporative layer 212A. In embodiments including the second nonwoven layer, fluids may flow through the holes 262A of the second support layer 210A and the holes 254A of the second filter carrier 208A to reach the second nonwoven layer. Once in contact with the second nonwoven layer, fluids may be distributed across the second nonwoven layer and may contact a maximum amount of the surface area of the second evaporative layer 212A. If more fluids are able to contact the second evaporative layer 212A, there may be an increase in the moisture vapor transmission rate of the canister 115. The second nonwoven layer may be comprised of a similar material as the first nonwoven layer 902.

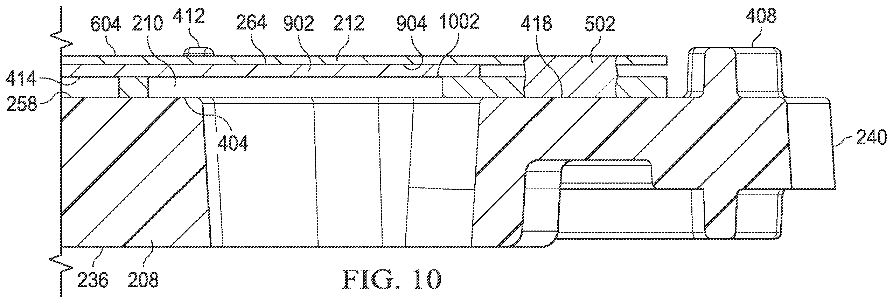

Figure 10 is a perspective view illustrating additional details that may be associated with the portion of the first canister section 202 of Figure 9. When the first canister section 202 is assembled, the first surface 258 of the first support layer 210 is adjacent to the second surface 404 of the first filter carrier 208, a first surface 1002 of the first nonwoven layer 902 may be adjacent to the second surface 414 of the first support layer 210, and the first surface 264 of the first evaporative layer 212 may be adjacent to the second surface 904 of the first nonwoven layer 902. The second surface 416 of the first evaporative layer 212 may face away from the first canister section 202. The fourth extension 412 and the second extension 408 may project from the second surface 404 of the first filter carrier 208 past the first support layer 210, the first nonwoven layer 902, and the first evaporative layer 212. The fourth extension 412 and the second extension 408 may be configured to couple to the second filter carrier 208A such that the fluid flow pathway 209 may be formed between the first canister section 202 and the second canister section 204.

The first filter carrier 208, the first support layer 210, and the first evaporative layer 212 may be coupled together by a weld 502. The weld may follow the connection path 418. The weld 502 may extend from the second surface 416 of the first evaporative layer 212, through the first support layer 210 and to the second surface 404 of the first filter carrier 208. In other embodiments, the first filter carrier 208, the first support layer 210, and the first evaporative layer 212 may be coupled by any of the methods described in detail above, such as adhering, bonding, friction coupling, etc. The first nonwoven layer 902 can be disposed between the first support layer 210 and the first evaporative layer 212. The first nonwoven layer 902 is excluded from the weld 502. Excluding the first nonwoven layer 902 from the weld 502 may help ensure that the weld 502 is not compromised. This may ensure that fluids stored in the first canister section 202 cannot escape from the canister 115 other than by being evaporated through the first evaporative layer 212.

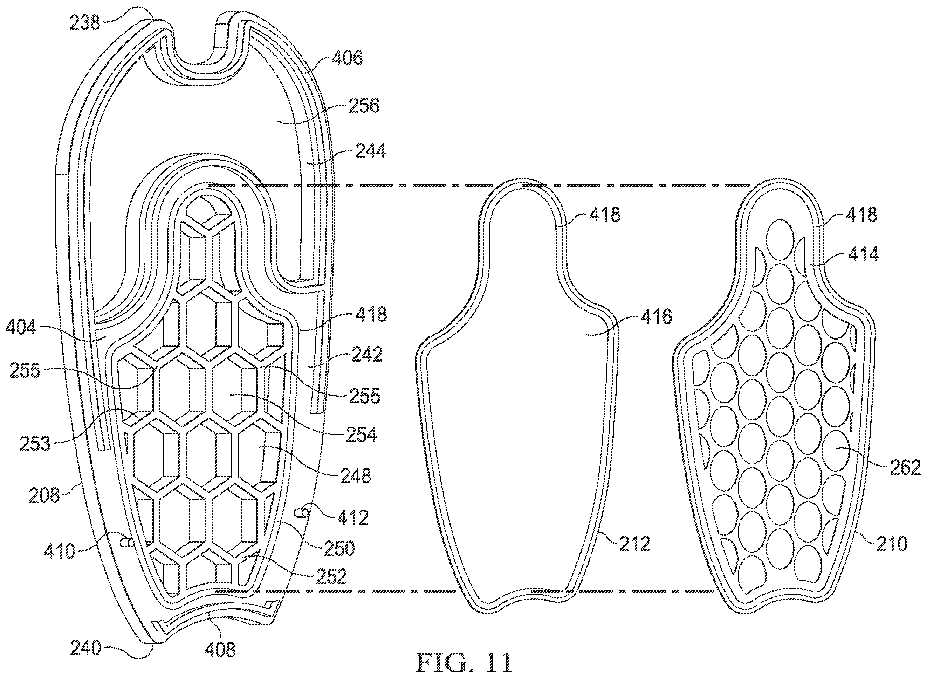

Figure 11 is an exploded view illustrating additional details that may be associated with some example embodiments of a portion of the first canister section 202. In the embodiment illustrated in Figure 11, the first canister section 202 may include a first filter carrier 208, a first evaporative layer 212, and a first support layer 210. The first surface 264 of the first evaporative layer 212 faces the second surface 404 of the first filter carrier 208 and the first surface 258 of the first support layer 210 faces the second surface 416 of the first evaporative layer 212. In the illustrative embodiment, fluid from the canister 115 may reach the first evaporative layer 212 before coming into contact with the first support layer 210. The first support layer 210 may be disposed between the first evaporative layer 212 and the fluid flow pathway 209 to provide stability to the first evaporative layer 212.

In some embodiments, the second canister section 204 may include a second filter carrier 208A, a second evaporative layer 212A, and a second support layer 210A. The first surface 264A of the second evaporative layer 212A faces the second surface of the second support layer 210A. The second surface of the second evaporative layer faces the first surface 236A of the second filter carrier 208A. When the second canister section 204 is organized with the second evaporative layer 212A disposed between the second filter carrier 208A and the second support layer 210A, fluid from the canister 115 may reach the second evaporative layer 212A before coming into contact with the second support layer 210A. The second support layer 210A may be disposed between the second evaporative layer 212A and the fluid flow pathway 209 to provide stability to the second evaporative layer 212A.

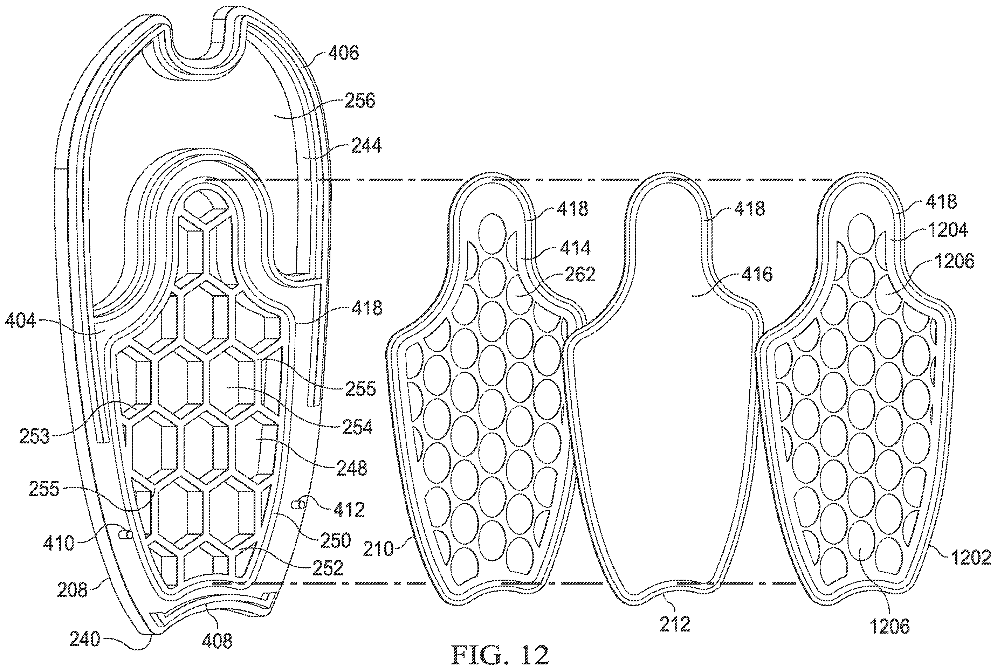

Figure 12 is an exploded view illustrating additional details that may be associated with some embodiments of a portion of the first canister section 202. In the embodiment illustrated in Figure 12, the first canister section 202 may further include a third support layer 1202. The third support layer 1202 may be disposed between the first evaporative layer 212 and the fluid flow pathway 209. The first surface 258 of the first support layer 210 faces the second surface 404 of the first filter carrier 208. The first surface 264 of the first evaporative layer 212 faces the second surface 414 of the first support layer 210. A first surface of the third support layer 1202 may face the second surface 416 of the first evaporative layer 212. A second surface 1204 of the third support layer 1202 may face away from the first canister section 202.

The third support layer 1202 may be perforated with a plurality of holes 1206. Each hole 1206 of the plurality of holes 1206 may have a circular shape. In other embodiments, the plurality of holes 1206 may comprise different sized and shapes. The plurality of holes 1206 may maintain all or substantially all of the surface area of the third support layer 1202. The plurality of holes 1206 of the third support layer 1202 may be configured to allow evaporated fluids from the canister 115 to escape the first canister section 202 to the fluid flow pathway 209. The third support layer 1202 may be substantially the same size and shape as the first support layer 210. The third support layer 1202 may provide an extra layer of protection to the first evaporative layer 212. This design may reduce the MVTR of the canister 115 but the canister 115 may be more structurally sound which may be desirable in certain situations.

The first filter carrier 208, the first support layer 210, the first evaporative layer 212, and the third support layer 1202 may be coupled along the connection path 418. The first filter carrier 208, the first support layer 210, the first evaporative layer 212, and the third support layer 1202 may be coupled by a weld, double sided adhesive, or compression gaskets as described above or in another way that creates a seal that ensures fluids from the dressing 110 will be contained inside the canister 115.

In some embodiments, the second canister section 204 may further include a fourth support layer disposed between the second evaporative layer 212A and the fluid flow pathway 209. The fourth support layer may have a first surface that may face the fluid flow pathway 209 and a second surface that may face the first surface 264A of the second evaporative layer 212A. The fourth support layer may be perforated with a plurality of holes. Each hole of the plurality of holes may have a circular shape. In other embodiments, the plurality of holes may comprise different sized and shapes. The plurality of holes may maintain all or substantially all of the surface area of the fourth support layer. The plurality of holes of the fourth support layer may be configured to allow evaporated fluids from the canister 115 to escape the second canister section 204 to the fluid flow pathway 209. The fourth support layer may be substantially the same size and shape as the second support layer 210A. The fourth support layer may provide an extra layer of protection to the second evaporative layer 212A. This design may reduce the MVTR of the canister 115 but the canister 115 may be more structurally sound which may be desirable in certain situations.

The second filter carrier 208A, the second support layer 210A, the second evaporative layer 212A, and the fourth support layer may be coupled along the connection path. The second filter carrier 208A, the second support layer 210A, the second evaporative layer 212A, and the fourth support layer may be coupled by a weld, double sided adhesive, or compression gaskets as described above or in another way that creates a seal that ensures fluids from the dressing 110 will be contained inside the canister 115.

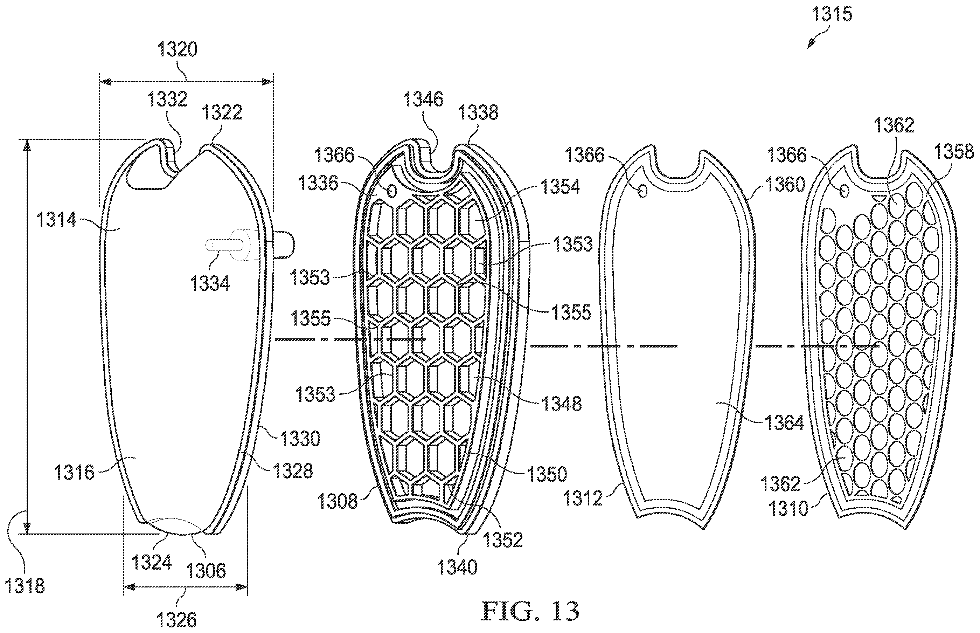

Figure 13 is an exploded view of an alternate embodiment of a canister 1315 that can be used with the therapy system 100 of Figure 1. Canister 1315 may include an outer section 1306, a filter carrier 1308, an evaporative layer 1312, and a support layer 1310. In some embodiments, the outer section 1306 may have a base 1314 with a first side or a first surface 1316 and a second side or a second surface opposite the first surface 1316. The first surface 1316 may have a generally convex shape and may face away from the filter carrier 1308, the evaporative layer 1312, and the support layer 1310. The second surface may be concave shaped and may face the filter carrier 1308, the evaporative layer 1312, and the support layer 1310. In an illustrative embodiment, the base 1314 may have a generally ovular shape having a flattened end. In other embodiments, the base 1314 may be rectangular, circular, triangular, ovular, or amorphous in shape. The base 1314 may have a length 1318, and a width 1320 at a first end 1322, the width 1320 may be perpendicular to the length 1318. There may be a second end 1324 opposite the first end 1322. The second end 1324 may have a width 1326. In some embodiments, the width 1320 may be greater than the width 1326. In other embodiments, the width 1320 may be substantially equal to the width 1326. In some embodiments, the length 1318 may be greater than the width 1320 and the width 1326. In other embodiments, the length 1318 may be substantially equal to the width 1320 and the width 1326.

The base 1314 may have a periphery or exterior edge 1328. An extension 1330 may extend from the exterior edge 1328 of the base 1314 towards the filter carrier 1308, the evaporative layer 1312, and the support layer 1310. The extension 1330 may be coincident with the exterior edge 1328 of the base 1314. The extension 1330 may extend from and be perpendicular to a plane defined by the exterior edge 1328 of the base 1314. In some embodiments, the exterior edge 1328 may be a joint of the extension 1330 and the base 1314. For example, the exterior edge 1328 may be formed by coupling the extension 1330 to the base 1314. In some embodiment, the exterior edge 1328 may comprise a chamfered or beveled edge. In other embodiments, the exterior edge 1328 may comprise a vertex of a perpendicular angle between the extension 1330 and the base 1314. In some embodiments, a receiver or a notch 1332 can be formed in the first end 1322. In some embodiments, the notch 1332 extends through the base 1314 from the first surface 1316 to the second surface. The notch 1332 may be positioned proximate a center of the width 1320. In other embodiments, the notch 1332 may not be centered on the width 1320. In some embodiments, the notch 1332 may have a width less than the width 1320.

A fluid inlet 1334 can be disposed in the outer section 1306. In some embodiments, the fluid inlet 1334 may provide fluid communication across the base 1314. In some embodiments, the fluid inlet 1334 may be configured to fluidly couple the canister 1315 to the dressing 110. For example, the fluid inlet 1334 may be a fluid port configured to receive a fluid conductor such as a tube having at least one lumen. The fluid conductor may be coupled to the fluid inlet 1334 and similarly coupled to the dressing 110. In this manner, the canister 1315 may be fluidly coupled to the dressing 110. In some embodiments, the fluid inlet 1334 may be disposed proximate the first end 1322 of the base 1314. In some embodiments, the fluid inlet 1334 may be positioned near a midpoint of the width 1320 at the first end 1322. In the illustrative embodiment, the fluid inlet 1334 may be separated from a center of the width 1320 of the first end 1322. In other embodiments, the fluid inlet 1334 may be disposed in other locations on the base 1314.

The filter carrier 1308 may comprise a first side or a first surface 1336 and a second side or a second surface opposite the first surface 1336. The filter carrier 1308 may have a first end 1338 and a second end 1340 opposite the first end 1338. The first end 1338 may have a width substantially equal to the width 1320 of the outer section 1306, and the second end 1340 may have a width substantially equal to the width 1326 of the outer section 1306. In other embodiments, the first end 1338 may have a width less than the width 1320, and the second end 1340 may have a width less than the width 1326. The filter carrier 1308 may have a length that is substantially equal to the length 1318 of the outer section 1306. In still other embodiments, the filter carrier 1308 may comprise a different size or shape.

The first surface 1336 of the filter carrier 1308 may face the outer section 1306 and the second surface of the filter carrier 1308 may face the support layer 1310. In some embodiments, a receiver or a notch 1346 can be formed in the first end 1338. In some embodiments, the notch 1346 extends through the filter carrier 1308 from the first surface 1336 to the second surface. The notch 1346 may be positioned proximate to a center of the width of the first end 1338. In other embodiments, the notch 1346 may not be centered on the width of the first end 1338. In some embodiments, the notch 1346 may have a width less than the width of the first end 1338. In some embodiments, the notch 1346 may be aligned or coincident with the notch 1332 of the outer section 1306.

The filter carrier 1308 may have a periphery or a peripheral portion, such as a filter boundary 1350 surrounding and defining an opening 1348. In some embodiments, the opening 1348 can comprise a substantial portion of the filter carrier 1308. For example, the opening 1348 may comprise greater than 50% of the surface area of the filter carrier 1308. In some embodiments, the opening 1348 may comprise about 50% to 70% of the surface area of the filter carrier 1308. In other embodiments, the opening 1348 may be about 90% or greater of the surface area of the filter carrier 1308. A support framework, such as a filter section 1352 can be disposed in the opening 1348.

The filter section 1352 can comprise a plurality of arms, beams, or braces 1353 extending across the opening 1348. In some embodiments, the filter section 1352 forms a plurality of holes 1354. Each hole 1354 of the plurality of holes 1354 may have a hexagonal shape. In some embodiments, each vertex 1355 of each hole 1354 may be proximate to at least one vertex 1355 of an adjacent hole 1354. In other embodiments, the plurality of holes 1354 may comprise different sizes and shapes. The plurality of holes 1354 may maintain all or substantially all of the opening 1348 of the filter carrier 1308. The plurality of holes 1354 of the filter section 1352 may be configured to allow fluid flow across the filter section 1352.

The filter carrier 1308 may be coupled to the outer section 1306. For example, the periphery of the first surface 1336 of the filter carrier 1308 may be coupled to the extension 1330 of the outer section 1306. In some embodiments, the filter carrier 1308 may be coupled to the outer section 1306 at one or more attachment points. In other embodiments, the filter carrier 1308 and the outer section 1306 may be coupled by compression gaskets, double sided adhesives, a weld, or any other suitable method of coupling to seal the filter carrier 1308 to the outer section 1306.

The evaporative layer 1312 may comprise a first side or a first surface 1364 and a second side or a second surface opposite the first surface 1364. The evaporative layer 1312 may comprise one or more evaporative membrane layers. The evaporative layer 1312 may be a continuous sheet having substantially the same size as the filter carrier 1308. The evaporative layer 1312 may be smaller in size than the filter carrier 1308 such that a periphery 1360 of the evaporative layer 1312 aligns with the filter boundary 1350 of the filter carrier 1308. The first surface 1364 of the evaporative layer 1312 may be configured to cover the opening 1348 of the filter carrier 1308. The first surface 1364 of the evaporative layer 1312 may be coupled to the second surface of the filter carrier 1308 along the filter boundary 1350.

The support layer 1310 may have a first side or a first surface 1358 and a second side or a second surface opposite the first surface 1358. The support layer 1310 may be substantially the same shape and size as the evaporative layer 1312. In other embodiments, the support layer 1310 may be differently shaped.

The support layer 1310 may be perforated with a plurality of holes 1362. Each hole 1362 of the plurality of holes 1362 may have a circular shape. In other embodiments, the plurality of holes 1362 may comprise different sizes and shapes. The plurality of holes 1362 may maintain all or substantially all of the surface area of the support layer 1310. The plurality of holes 1362 of the support layer 1310 may be configured to allow fluid flow across the support layer 1310.

A negative-pressure inlet 1366 can be disposed in the support layer 1310, the evaporative layer 1312, and the filter carrier 1308. There may be a hydrophobic filter disposed in the negative-pressure inlet 1366 in order to prevent ingress of liquids into the negative-pressure source 105. The negative-pressure inlet 1366 may be substantially centered with respect to the length but may be off-centered with respect to the width of the support layer 1310, the evaporative layer 1312, and the filter carrier 1308. In other embodiments, the negative-pressure inlet 1366 may be substantially centered with respect to both the length and the width or may be located in another area in the support layer 1310, the evaporative layer 1312, and the filter carrier 1308. In some embodiments, the negative-pressure inlet 1366 may be configured to fluidly couple the canister 1315 to the negative-pressure source 105. For example, a fluid conductor may be coupled to the negative-pressure inlet 1366 and similarly coupled to the negative-pressure source 105 to fluidly couple the canister 1315 to the negative-pressure source 105.

In some embodiments not pictured herein, there may be a second support layer disposed between the evaporative layer 1312 and the filter carrier 1308. The second support layer may be substantially the same shape and size as the support layer 1310. The second support layer may provide additional structural support for the evaporative layer 1312 similar to the portion of the first canister section 202 shown in Figure 12.

A first step in assembling the canister 1315 may be to couple the support layer 1310, the evaporative layer 1312, and the filter carrier 1308. In some embodiments, the evaporative layer 1312 and the support layer 1310 may be welded to the filter carrier 1308 along the filter boundary 1350. A weld may extend from the second surface of the support layer 1310, through the evaporative layer 1312, and to the second surface of the filter carrier 1308. In other embodiments, the support layer 1310, the evaporative layer 1312, and the filter carrier 1308 may be coupled by other methods such as adhesives, compression gaskets, or other attachment methods. Once the support layer 1310, the evaporative layer 1312, and the filter carrier 1308 are coupled, the filter carrier 1308 may be coupled to the outer section 1306. In some embodiments, the periphery of the first surface 1336 of the filter carrier 1308 may be coupled to the extension 1330 of the outer section 1306. In some embodiments, the filter carrier 1308 may be coupled to the outer section 1306 at one or more attachment points. In other embodiments, the filter carrier 1308 and the outer section 1306 may be coupled by compression gaskets, double sided adhesives, a weld, or any other suitable method of coupling to seal the filter carrier 1308 to the outer section 1306.

The canister 1315 may collect fluids from the dressing 110 in a chamber formed between the outer section 1306 and the filter carrier 1308. Fluids in contact with the evaporative layer 1312 may transfer through the evaporative layer 1312 by osmosis to reach ambient environment surrounding the canister 1315. The support layer 1310 may provide support for the evaporative layer 1312 to protect the evaporative layer 1312 from the ambient environment. In some embodiments, the support layer 1310 may be located proximate to the therapy unit 145 to provide more protection to the evaporative layer 1312. Locating the support layer 1310 near the therapy unit 145 may substantially prevent contact from the ambient environment with the evaporative layer 1312 so ensure the liquid-impermeable, vapor-permeable properties of the evaporative layer 1312.

The outer section 1306 and the filter carrier 1308 may comprise a type of material having sufficient rigidity and structural integrity to withstand the reduced pressure required for negative-pressure treatment and to contain fluid therein. In some embodiments, the outer section 1306 and the filter carrier 1308 may comprise a clear rigid plastic such as ABS or TPU. Some exemplary materials of the outer section 1306 and the filter carrier 1308 are plastics, polymers, thermoplastics, metals, metal alloys, composition material, fiber-type materials, and other similar materials. The plastics described herein may be a substance or structure capable of being shaped or molded with or without the application of heat, a high polymer, usually synthetic, combined with other ingredients such as curatives, fillers, reinforcing agents, plasticizers, etc. Plastics can be formed or molded under heat and pressure in its raw state and machined to high dimensional accuracy, trimmed and finished in its hardened state. The thermoplastic type can be resoftened to its original condition by heat. In addition, the plastics may mean engineered plastics such as those that are capable of sustaining high levels of stress and are machinable and dimensionally stable. Some exemplary plastics are nylon, acetyls, polycarbonates, ABS resins, PPO/styrene, Isoplast 2530, TURLUX HS 2822, and polybutylene terephthalate. The thermoplastics described herein may be high polymers that soften when exposed to heat and return to their original condition when cooled to room temperature.

The evaporative layer 1312 and the support layer 1310 may be comprised of a thermoplastic polyurethane (TPU) material. In some embodiments, the TPU material may be a high moisture vapor transmission rate (MVTR) film. The high MVTR film should have an MVTR of about 2000 to 5000 g/m²/24hrs in an upright cup test. The evaporative layer 1312 may be about 15µm -50µm thick. In some embodiments, the evaporative layer 1312 may include two layer of high MVTR film that may each be about 20µm thick. In other embodiments, the evaporative layer 1312 may comprise one layer of high MVRT film that is about 40µm thick. The support layer 1310 may be about 50µm -500µm thick. In some optimal embodiments, the support layer 1310 may be about 250µm thick. In some embodiments, the high MVTR film may be BASF E1385A 12000. In other embodiments, the high MVTR film may be COVESTRO VPT 9121, or COVESTRO Platilon U 250µm.

The systems, apparatuses, and methods described herein may provide significant advantages. For example, the structure of the canister 115 may improve manufacturing yield and performance of the canister 115. When the first nonwoven layer 902 is included in the first canister section 202, elimination of the first nonwoven layer 902 from the connection path 418 may improve the reliability of the sealing methods applied between the first filter carrier 208, the first support layer 210, and the first evaporative layer 212. The embodiments described herein may also allow for the first evaporative layer 212 and the second evaporative layer 212A to be as thin as possible which may help to increase the MVTR of the canister 115. These embodiments may also reduce the components used to create the canister 115 when the first nonwoven layer 902 and the second nonwoven layer are not included. The embodiments described herein can produce canisters that reduce material variations, reduce waste, increase patient quality of life, and reduce the cost of production.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 110, the canister 115, or both may be eliminated or separated from other components for manufacture or sale. In other example configurations, the controller 130 may also be manufactured, configured, assembled, or sold independently of other components.

## Claims

1. A canister for use in negative-pressure therapy comprising:
a first wall (206);
a second wall (206A) coupled to the first wall (206);
a chamber disposed between the first wall (206) and the second wall (206A), the chamber configured to be sealed from an ambient environment and to store fluid;
a pathway (209) disposed between the first wall (206) and the second wall (206A), the pathway (209) configured to be open to the ambient environment; and
a barrier (212) separating the pathway (209) from the chamber, the barrier (212, 212A) configured to permit evaporated fluid in the chamber to transmit through the barrier (212, 212A) into the pathway (209);
wherein the barrier (212, 212A) comprises:
a first barrier wall (212) comprising one or more layers; and
a second barrier (212A) wall comprising one or more layers
wherein the pathway is formed by:
coupling the first barrier wall (212) to the first wall (206);
coupling the second barrier wall (212A) to the second wall (206A); and
coupling the first wall (206) to the second wall (206A) so that the first barrier wall (212) and the second barrier wall (212A) face each other and form a boundary between the pathway (209) and the chamber, the pathway (209) being defined partly between the facing barrier walls (212, 212A); and
wherein the chamber is defined by the first wall (206), second wall (206A) first barrier wall (212), and second barrier wall (212A), wherein
the one or more layers of the first barrier wall (212) comprise:
a first carrier layer (208) comprising a first side and a second side;
a first support layer (210) comprising a first side and a second side, the first support layer (210) coupled to the first carrier layer (208) so that the first side of the first support layer (210) is proximate to the second side of the first carrier layer (208); and
a first evaporative membrane layer (212) comprising a first side and a second side, the first evaporative membrane layer (212) coupled to the first support layer (210) so that the first side of the first evaporative membrane layer (212) is proximate to the second side of the first support layer (210);
the one or more layers of the second barrier wall (212A) comprise:
a second carrier layer (208A) comprising a first side and a second side;
a second support layer (210A) comprising a first side and a second side, the second support layer (210A) coupled to the second carrier layer (208A) so that the second side of the second support layer (210A) is proximate to the first side of the second carrier layer (208A); and
a second evaporative membrane layer (212A) comprising a first side and a second side, the second evaporative membrane layer (212A) coupled to the second support layer (210A) so that the second side of the second evaporative membrane layer (212A) is proximate to the first side of the second support layer (210A).

2. The canister of claim 1, wherein:
the first carrier layer (208) comprises:
a first section (244) having an opening (256); and
a second section (242) having a plurality of holes (248);
the second carrier layer (208A)comprises:
a first section (244A) having an opening (256A); and
a second section (242A) having a plurality of holes (248A).

3. The canister of claim 2, further comprising:
a first weld coupling the first support layer (210) to the second side of the first carrier layer (208) and the first evaporative membrane layer (212) to the second side of the first support layer (210), the first weld aligned with a periphery of the plurality of holes (248) of the second section (242) of the first carrier layer (208); and
a second weld coupling the second support layer (210A) to the first side of the second carrier layer (208A) and the second evaporative membrane layer (212A) to the first side of the second support layer (210A), the second weld aligned with a periphery of the plurality of holes (248A) of the second section of the second carrier layer (208A).

4. The canister of claim 2, wherein:
the first support layer (210) is adhered to the second side of the first carrier layer (208) along a periphery of the plurality of holes (248) of the second section (242) of the first carrier layer (208);
the first evaporative membrane layer (212) is adhered to the second side of the first support layer (210) along the periphery of the plurality of holes (248) of the second section (242) of the first carrier layer (208);
the second support layer (210A) is adhered to the first side of the second carrier layer (208A) along a periphery of the plurality of holes of the second section (242A) of the second carrier layer (208A); and
the second evaporative membrane layer (212A) is adhered to the first side of the second support layer (210A) along the periphery of the plurality of holes of the second section (242A) of the second carrier layer (208A).

5. The canister of claim 2, wherein each hole of the plurality of holes (248, 248A) of the second section (242, 242A) of the first carrier layer (208) and the second carrier layer (208A) having a hexagonal shape, each vertex of each hole proximate to at least one vertex of an adjacent hole.

6. The canister of claim 5, wherein each of the first support layer (210) and the second support layer (210A) has a plurality of holes (262, 262A) extending through the first support layer (210) and the second support layer (210A).

7. The canister of claim 6, wherein each hole of the plurality of holes (262, 262A) of the first support layer (210) and the second support layer (210A) having a hexagonal shape, each vertex of each hole proximate to at least one vertex of an adjacent hole.

8. The canister of claim 7, wherein:
the plurality of holes (262) of the first support layer (212) being aligned with the plurality of holes (248) of the first carrier layer (208); and
at least one spot weld joining the first support layer (210) to the first carrier layer (208) at at least one vertex of the aligned plurality of holes (248, 262); and
the plurality of holes (262A) of the second support layer (212A) being aligned with the plurality of holes (248A) of the second carrier layer (208A); and
at least one spot weld joining the second support layer (212A) to the second carrier layer (208A) at at least one vertex of the aligned plurality of holes (248A, 262A).

9. The canister of claim 1, wherein:
the first support layer (212) comprises thermoplastic polyurethane, TPU; and
the second support (212A) layer comprises TPU.

10. The canister of claim 1 wherein the first evaporative membrane layer (212) and the second evaporative membrane layer (212A) are between about 15 µm and 30 µm

11. The canister of claim 1, wherein the first support layer (210) and the second support layer (210A) are between about 50 µm and 500 µm.

12. The canister of claim 1, wherein the first support layer and the second support layer are about 250 µm.

13. The canister of claim 1, wherein:
The first barrier wall (212) further comprises a first nonwoven layer with a first side and a second side, the first nonwoven layer coupled to the first support layer (210) and the first evaporative membrane layer (212) so that the first side of the first nonwoven layer is proximate to the second side of the first support layer (210) and the second side of the first nonwoven layer is proximate to the first side of the first evaporative membrane layer (212); and
the second barrier wall (212A) further comprises a second nonwoven layer with a first side and a second side, the second nonwoven layer coupled to the second support layer (210A) and the second evaporative membrane layer (212A) so that the first side of the second nonwoven layer is proximate to the second side of the second evaporative membrane layer (212A) and the second side of the second nonwoven layer is proximate to the first side of the second support layer (210A).

## Patentansprüche

1. Ein Kanister zur Verwendung in einer Unterdrucktherapie, aufweisend:
eine erste Wand (206);
eine zweite Wand (206A), die mit der ersten Wand (206) gekoppelt ist;
eine Kammer, die zwischen der ersten Wand (206) und der zweiten Wand (206A) angeordnet ist, wobei die Kammer konfiguriert ist, um von einer Umgebungsatmosphäre abgedichtet zu sein und Fluid zu speichern;
einen Pfad (209), der zwischen der ersten Wand (206) und der zweiten Wand (206A) angeordnet ist, wobei der Pfad (209) konfiguriert ist, um zu der Umgebungsatmosphäre hin offen zu sein; und
eine Sperre (212), die den Pfad (209) von der Kammer trennt, wobei die Sperre (212, 212A) konfiguriert ist, um verdampftem Fluid in der Kammer zu ermöglichen, durch die Sperre (212, 212A) in den Pfad (209) zu transmittieren;
wobei die Sperre (212, 212A) aufweist:
eine erste Sperrenwand (212), aufweisend eine oder mehrere Schichten; und
eine zweite Sperren(212A)-Wand, aufweisend eine oder mehrere Schichten,
wobei der Pfad ausgebildet wird durch:
Koppeln der ersten Sperrenwand (212) mit der ersten Wand (206);
Koppeln der zweiten Sperrenwand (212A) mit der zweiten Wand (206A); und
Koppeln der ersten Wand (206) mit der zweiten Wand (206A), sodass die erste Sperrenwand (212) und die zweite Sperrenwand (212A) einander zugewandt sind und eine Grenze zwischen dem Pfad (209) und der Kammer ausbilden, wobei der Pfad (209) zwischen den zugewandten Sperrenwänden (212, 212A) teilweise definiert ist; und
wobei die Kammer durch die erste Wand (206), die zweite Wand (206A), die erste Sperrenwand (212) und die zweite Sperrenwand (212A) definiert ist, wobei die eine oder die mehreren Schichten der ersten Sperrenwand (212) aufweisen:
eine erste Trägerschicht (208), aufweisend eine erste Seite und eine zweite Seite;
eine erste Stützschicht (210), aufweisend eine erste Seite und eine zweite Seite, wobei die erste Stützschicht (210) mit der ersten Trägerschicht (208) gekoppelt ist, sodass die erste Seite der ersten Stützschicht (210) nahe der zweiten Seite der ersten Trägerschicht (208) liegt; und
eine erste Verdampfungsmembranschicht (212), aufweisend eine erste Seite und eine zweite Seite, wobei die erste Verdampfungsmembranschicht (212) mit der ersten Stützschicht (210) gekoppelt ist, sodass die erste Seite der ersten Verdampfungsmembranschicht (212) nahe der zweiten Seite der ersten Stützschicht (210) liegt;
die eine oder die mehreren Schichten der zweiten Sperrenwand (212A) aufweisen:
eine zweite Trägerschicht (208A), aufweisend eine erste Seite und eine zweite Seite;
eine zweite Stützschicht (210A), aufweisend eine erste Seite und eine zweite Seite, wobei die zweite Stützschicht (210A) mit der zweiten Trägerschicht (208A) gekoppelt ist, sodass die zweite Seite der zweiten Stützschicht (210A) nahe der ersten Seite der zweiten Trägerschicht (208A) liegt; und
eine zweite Verdampfungsmembranschicht (212A), aufweisend eine erste Seite und eine zweite Seite, wobei die zweite Verdampfungsmembranschicht (212A) mit der zweiten Stützschicht (210A) gekoppelt ist, sodass die zweite Seite der zweiten Verdampfungsmembranschicht (212A) nahe der ersten Seite der zweiten Stützschicht (210A) liegt.

2. Der Kanister nach Anspruch 1, wobei:
die erste Trägerschicht (208) aufweist:
einen ersten Bereich (244), der über eine Öffnung (256) verfügt; und
einen zweiten Bereich (242), der über eine Mehrzahl von Löchern (248) verfügt;
die zweite Trägerschicht (208A) aufweist:
einen ersten Bereich (244A), der über eine Öffnung (256A) verfügt; und
einen zweiten Bereich (242A), der über eine Mehrzahl von Löchern (248A) verfügt.

3. Der Kanister nach Anspruch 2, ferner aufweisend:
eine erste Schweißung, die die erste Stützschicht (210) mit der zweiten Seite der ersten Trägerschicht (208) und die erste Verdampfungsmembranschicht (212) mit der zweiten Seite der ersten Stützschicht (210) koppelt, wobei die erste Schweißung an einem Umfang der Mehrzahl von Löchern (248) des zweiten Bereichs (242) der ersten Trägerschicht (208) ausgerichtet ist; und
eine zweite Schweißung, die die zweite Stützschicht (210A) mit der ersten Seite der zweiten Trägerschicht (208A) und die zweite Verdampfungsmembranschicht (212A) mit der ersten Seite der zweiten Stützschicht (210A) koppelt, wobei die zweite Schweißung an einem Umfang der Mehrzahl von Löchern (248A) des zweiten Bereichs der zweiten Trägerschicht (208A) ausgerichtet ist.

4. Der Kanister nach Anspruch 2, wobei:
die erste Stützschicht (210) mit der zweiten Seite der ersten Trägerschicht (208) entlang eines Umfangs der Mehrzahl von Löchern (248) des zweiten Bereichs (242) der ersten Trägerschicht (208) verklebt ist;
die erste Verdampfungsmembranschicht (212) mit der zweiten Seite der ersten Stützschicht (210) entlang des Umfangs der Mehrzahl von Löchern (248) des zweiten Bereichs (242) der ersten Trägerschicht (208) verklebt ist;
die zweite Stützschicht (210A) mit der ersten Seite der zweiten Trägerschicht (208A) entlang eines Umfangs der Mehrzahl von Löchern des zweiten Bereichs (242A) der zweiten Trägerschicht (208A) verklebt ist; und
die zweite Verdampfungsmembranschicht (212A) mit der ersten Seite der zweiten Stützschicht (210A) entlang des Umfangs der Mehrzahl von Löchern des zweiten Bereichs (242A) der zweiten Trägerschicht (208A) verklebt ist.

5. Der Kanister nach Anspruch 2, wobei jedes Loch der Mehrzahl von Löchern (248, 248A) des zweiten Bereichs (242, 242A) der ersten Trägerschicht (208) und der zweiten Trägerschicht (208A) über eine hexagonale Form verfügt, wobei jeder Eckpunkt jedes Lochs nahe mindestens einem Eckpunkt eines angrenzenden Lochs liegt.

6. Der Kanister nach Anspruch 5, wobei jede der ersten Stützschicht (210) und der zweiten Stützschicht (210A) über eine Mehrzahl von Löchern (262, 262A), die sich durch die erste Stützschicht (210) und die zweite Stützschicht (210A) erstrecken, verfügt.

7. Der Kanister nach Anspruch 6, wobei jedes Loch der Mehrzahl von Löchern (262, 262A) der ersten Stützschicht (210) und der zweiten Stützschicht (210A) über eine hexagonale Form verfügt, wobei jeder Eckpunkt jedes Lochs nahe mindestens einem Eckpunkt eines angrenzenden Lochs liegt.

8. Der Kanister nach Anspruch 7, wobei:
die Mehrzahl von Löchern (262) der ersten Stützschicht (212) an der Mehrzahl von Löchern (248) der ersten Trägerschicht (208) ausgerichtet ist; und
mindestens eine Punktschweißung die erste Stützschicht (210) mit der ersten Trägerschicht (208) an mindestens einem Eckpunkt der ausgerichteten Mehrzahl von Löchern (248, 262) verbindet; und
die Mehrzahl von Löchern (262A) der zweiten Stützschicht (212A) an der Mehrzahl von Löchern (248A) der zweiten Trägerschicht (208A) ausgerichtet ist; und
mindestens eine Punktschweißung die zweite Stützschicht (212A) mit der zweiten Trägerschicht (208A) an mindestens einem Eckpunkt der ausgerichteten Mehrzahl von Löchern (248A, 262A) verbindet.

9. Der Kanister nach Anspruch 1, wobei:
die erste Stützschicht (212) thermoplastisches Polyurethan, TPU, aufweist; und
die zweite Stütz(212A)-Schicht TPU aufweist.

10. Der Kanister nach Anspruch 1, wobei die erste Verdampfungsmembranschicht (212) und die zweite Verdampfungsmembranschicht (212A) zwischen etwa 15 µm und 30 µm sind.

11. Der Kanister nach Anspruch 1, wobei die erste Stützschicht (210) und die zweite Stützschicht (210A) zwischen etwa 50 µm und 500 µm sind.

12. Der Kanister nach Anspruch 1, wobei die erste Stützschicht und die zweite Stützschicht etwa 250 µm sind.

13. Der Kanister nach Anspruch 1, wobei:
die erste Sperrenwand (212) ferner eine erste Vliesschicht mit einer ersten Seite und einer zweiten Seite aufweist, wobei die erste Vliesschicht mit der ersten Stützschicht (210) und der ersten Verdampfungsmembranschicht (212) gekoppelt ist, sodass die erste Seite der ersten Vliesschicht nahe der zweiten Seite der ersten Stützschicht (210) ist und die zweite Seite der ersten Vliesschicht nahe der ersten Seite der ersten Verdampfungsmembranschicht (212) ist; und
die zweite Sperrenwand (212A) ferner eine zweite Vliesschicht mit einer ersten Seite und einer zweiten Seite aufweist, wobei die zweite Vliesschicht mit der zweiten Stützschicht (210A) und der zweiten Verdampfungsmembranschicht (212A) gekoppelt ist, sodass die erste Seite der zweiten Vliesschicht nahe der zweiten Seite der zweiten Verdampfungsmembranschicht (212A) ist und die zweite Seite der zweiten Vliesschicht nahe der ersten Seite der zweiten Stützschicht (210A) ist.

## Revendications

1. Cartouche pour utilisation dans une thérapie par pression négative comprenant :
une première paroi (206) ;
une seconde paroi (206A) accouplée à la première paroi (206) ;
une chambre disposée entre la première paroi (206) et la seconde paroi (206A), la chambre étant conçue pour être scellée par rapport à l'environnement ambiant et pour stocker un fluide ;
une voie de passage (209) disposée entre la première paroi (206) et la seconde paroi (206A), la voie de passage (209) étant conçue pour être ouverte vers l'environnement ambiant ; et
une barrière (212) séparant la voie de passage (209) par rapport à la chambre, la barrière (212, 212A) étant conçue pour permettre à un fluide évaporé dans la chambre de se transmettre à travers la barrière (212, 212A) dans la voie de passage (209) ;
dans laquelle la barrière (212, 212A) comprend :
une première paroi de barrière (212) comprenant une ou plusieurs couches ; et
une seconde paroi de barrière (212A) comprenant une ou plusieurs couches
dans laquelle la voie de passage est formée par :
accouplement de la première paroi de barrière (212) à la première paroi (206) ;
accouplement de la seconde paroi de barrière (212A) à la seconde paroi (206A) ; et
accouplement de la première paroi (206) à la seconde paroi (206A) de sorte que la première paroi de barrière (212) et la seconde paroi de barrière (212A) se font face l'une l'autre et forment une limite entre la voie de passage (209) et la chambre, la voie de passage (209) étant définie partiellement entre les parois de barrière (212, 212A) se faisant face ; et
dans laquelle la chambre est définie par la première paroi (206), la seconde paroi (206A), la première paroi de barrière (212) et la seconde paroi de barrière (212A), dans laquelle la ou les couches de la première paroi de barrière (212) comprennent :
une première couche porteuse (208) comprenant un premier côté et un second côté ;
une première couche de support (210) comprenant un premier côté et un second côté, la première couche de support (210) étant accouplée à la première couche porteuse (208) de sorte que le premier côté de la première couche de support (210) est à proximité du second côté de la première couche porteuse (208) ; et
une première couche de membrane évaporative (212) comprenant un premier côté et un second côté, la première couche de membrane évaporative (212) étant accouplée à la première couche de support (210) de sorte que le premier côté de la première couche de membrane évaporative (212) est à proximité du second côté de la première couche de support (210) ;
la ou les couches de la seconde paroi de barrière (212A) comprennent :
une seconde couche porteuse (208A) comprenant un premier côté et un second côté ;
une seconde couche de support (210A) comprenant un premier côté et un second côté, la seconde couche de support (210A) étant accouplée à la seconde couche porteuse (208A) de sorte que le second côté de la seconde couche de support (210A) est à proximité du premier côté de la seconde couche porteuse (208A) ; et
une seconde couche de membrane évaporative (212A) comprenant un premier côté et un second côté, la seconde couche de membrane évaporative (212A) étant accouplée à la seconde couche de support (210A) de sorte que le second côté de la seconde couche de membrane évaporative (212A) est à proximité du premier côté de la seconde couche de support (210A).

2. Cartouche selon la revendication 1, dans laquelle :
la première couche porteuse (208) comprend :
une première section (244) ayant une ouverture (256) ; et
une seconde section (242) ayant une pluralité de trous (248) ;
la seconde couche porteuse (208A) comprend :
une première section (244A) ayant une ouverture (256A) ; et
une seconde section (242A) ayant une pluralité de trous (248A).

3. Cartouche selon la revendication 2, comprenant en outre :
une première soudure accouplant la première couche de support (210) au second côté de la première couche porteuse (208) et la première couche de membrane évaporative (212) au second côté de la première couche de support (210), la première soudure étant alignée avec une périphérie de la pluralité de trous (248) de la seconde section (242) de la première couche porteuse (208) ; et
une seconde soudure accouplant la seconde couche de support (210A) au premier côté de la seconde couche porteuse (208A) et la seconde couche de membrane évaporative (212A) au premier côté de la seconde couche de support (210A), la seconde soudure étant alignée avec une périphérie de la pluralité de trous (248A) de la seconde section de la seconde couche porteuse (208A).

4. Cartouche selon la revendication 2, dans laquelle :
la première couche de support (210) est fixée par adhérence au second côté de la première couche porteuse (208) le long d'une périphérie de la pluralité de trous (248) de la seconde section (242) de la première couche porteuse (208) ;
la première couche de membrane évaporative (212) est fixée par adhérence au second côté de la première couche de support (210) le long de la périphérie de la pluralité de trous (248) de la seconde section (242) de la première couche porteuse (208) ;
la seconde couche de support (210A) est fixée par adhérence au premier côté de la seconde couche porteuse (208A) le long d'une périphérie de la pluralité de trous de la seconde section (242A) de la seconde couche porteuse (208A) ; et
la seconde couche de membrane évaporative (212A) est fixée par adhérence au premier côté de la seconde couche de support (210A) le long de la périphérie de la pluralité de trous de la seconde section (242A) de la seconde couche porteuse (208A).

5. Cartouche selon la revendication 2, dans laquelle chaque trou de la pluralité de trous (248, 248A) de la seconde section (242, 242A) de la première couche porteuse (208) et de la seconde couche porteuse (208A) a une forme hexagonale, chaque sommet de chaque trou étant à proximité d'au moins un sommet d'un trou adjacent.

6. Cartouche selon la revendication 5, dans laquelle chacune de la première couche de support (210) et de la seconde couche de support (210A) a une pluralité de trous (262, 262A) s'étendant à travers la première couche de support (210) et la seconde couche de support (210A).

7. Cartouche selon la revendication 6, dans laquelle chaque trou de la pluralité de trous (262, 262A) de la première couche de support (210) et de la seconde couche de support (210A) a une forme hexagonale, chaque sommet de chaque trou étant à proximité d'au moins un sommet d'un trou adjacent.

8. Cartouche selon la revendication 7, dans laquelle :
la pluralité de trous (262) de la première couche de support (212) sont alignés avec la pluralité de trous (248) de la première couche porteuse (208) ; et
au moins un point de soudure relie la première couche de support (210) à la première couche porteuse (208) au niveau d'au moins un sommet de la pluralité alignée de trous (248, 262) ; et
la pluralité de trous (262A) de la seconde couche de support (212A) sont alignés avec la pluralité de trous (248A) de la seconde couche porteuse (208A) ; et
au moins un point de soudure relie la seconde couche de support (212A) à la seconde couche porteuse (208A) au niveau d'au moins un sommet de la pluralité alignée de trous (248A, 262A).

9. Cartouche selon la revendication 1, dans laquelle :
la première couche de support (212) comprend du polyuréthane thermoplastique, TPU ; et
la seconde couche de support (212A) comprend du TPU.

10. Cartouche selon la revendication 1 dans laquelle la première couche de membrane évaporative (212) et la seconde couche de membrane évaporative (212A) font entre environ 15 µm et 30 µm.

11. Cartouche selon la revendication 1, dans laquelle la première couche de support (210) et la seconde couche de support (210A) font entre environ 50 µm et 500 µm.

12. Cartouche selon la revendication 1, dans laquelle la première couche de support et la seconde couche de support font environ 250 µm.

13. Cartouche selon la revendication 1, dans laquelle :
la première paroi de barrière (212) comprend en outre une première couche de non-tissé avec un premier côté et un second côté, la première couche de non-tissé étant accouplée à la première couche de support (210) et à la première couche de membrane évaporative (212) de sorte que le premier côté de la première couche de non-tissé est à proximité du second côté de la première couche de support (210) et le second côté de la première couche de non-tissé est à proximité du premier côté de la première couche de membrane évaporative (212) ; et
la seconde paroi de barrière (212A) comprend en outre une seconde couche de non-tissé avec un premier côté et un second côté, la seconde couche de non-tissé étant accouplée à la seconde couche de support (210A) et à la seconde couche de membrane évaporative (212A) de sorte que le premier côté de la seconde couche de non-tissé est à proximité du second côté de la seconde couche de membrane évaporative (212A) et le second côté de la seconde couche de non-tissé est à proximité du premier côté de la seconde couche de support (210A).
